# EUROPEAN PATENT SPECIFICATION

(11) **EP 2 609 846 B1**
(45) Date of publication and mention of the grant of the patent: **13.07.2016**
(21) Application number: 11844485.0
(22) Date of filing: 31.10.2011
(51) Int. Cl.: A61B 1/00, A61B 1/04

(54) **CAPSULE MEDICAL APPARATUS AND METHOD FOR MANUFACTURING THE SAME**
EINGEKAPSELTE MEDIZINISCHE VORRICHTUNG UND HERSTELLUNGSVERFAHREN DAFÜR
DISPOSITIF MÉDICAL À CAPSULE ET SON PROCÉDÉ DE FABRICATION

(30) Priority: 29.11.2010 JP 2010265796
(43) Date of publication of application: 03.07.2013
(73) Proprietor: Olympus Corporation, Shibuya-ku Tokyo (JP)
(72) Inventor: SEGAWA, Hidetake, Tokyo 151-0072 (JP)
(74) Representative: Gunzelmann, Rainer
(86) International application number: PCT/JP2011/075070
(87) International publication number: WO 2012/073634

(56) References cited:
- WO-A1-2005/065526
- WO-A1-2010/067765
- JP-A- 2006 141 897
- JP-A- 2006 149 462
- JP-A- 2006 192 252
- JP-A- 2006 247 081
- JP-A- 2008 006 301
- JP-A- 2008 183 455
- JP-A- 2008 264 083
- JP-A- 2008 272 439
- JP-A- 2008 278 962
- JP-A- 2009 018 077
- JP-A- 2009 060 925
- JP-A- 2009 125 101
- JP-A- 2010 069 209
- US-A1- 2004 027 459
- US-A1- 2007 118 012
- US-A1- 2010 174 141
- ANONYMOUS: 'Injection molding - Wikipedia, the free encyclopedia', [Online] 23 November 2010, XP055126598 Retrieved from the Internet: <URL:http://en.wikipedia.org/w/index.php?ti tle=Injection_molding&oldid=398416862> [retrieved on 2014-07-03]

## Description

### TECHNICAL FIELD

The present invention relates to a capsule medical apparatus that is inserted to an inside of a subject and captures an in-vivo image.

### BACKGROUND ART

In the field of an endoscope, a capsule medical apparatus provided with an imaging function and a wireless communication function in an inside of a casing having a capsule shape has appeared. In general, a capsule medical apparatus is inserted to an inside of a subject and obtains information, including a captured image and the like, concerning the subject from the inside of the subject.

Conventionally, such a capsule medical apparatus is assembled by filing and hardening an adhesive agent in a plurality of boards on which an imaging element, a wireless module, and the like are mounted to make the plurality of boards into a block shape with inter-board intervals kept and inserting the blocked boards into the capsule-shaped casing (see Patent Document 1).

Patent Document 1: Japanese Patent Application Laid-Open No. 2005-205071
Patent Document 2: Japanese Patent Application Laid-Open No. 2008-272439

US 2007/118012 A1 discloses an in-vivo imaging device having a closed housing containing internal components including two optical heads located on rigid portions of a circuit board connected by a flexible portion. The housing consists of a connecting sleeve located between two domes. The in-vivo imaging device is assembled by positioning at least part of the circuit board inside the connecting sleeve and folding the circuit board so that the optical heads cover ends of the connecting sleeve. The domes are placed over the optical heads and joined to the connecting sleeve.

US 2010/174141 A1 discloses a sleeve for simple assembly of in-vivo devices, such as endoscopy capsules. The sleeve comprises grippers and leaf springs at either end to hold the rigid portions of a rigid-flex PCB (printed circuit board) in a folded configuration before the PCB is inserted into an in-vivo device's housing. A method of assembly of the rigid-flex PCB into the sleeve is also disclosed.

### DISCLOSURE OF THE INVENTION

### PROBLEM TO BE SOLVED BY THE INVENTION

However, in the case of making boards and the like into block shape by an adhesive agent as explained above, an assembling process is complicated and requires a long time. Besides, while a method for assembling boards and the like by using a spacer is disclosed as another assembling method in Patent Document 2, a detail of the assembling is not disclosed.

The present invention has been achieved in view of the foregoing and an object of the present invention is to provide a configuration of a capsule medical apparatus which can be assembled easily and in a time shorter than the conventional technique.

### MEANS FOR SOLVING PROBLEM

To solve the problems explained above and to achieve the object, a capsule medical apparatus according to the present invention includes: the feature of claim 1.

In the capsule medical apparatus, a battery segment that elastically deforms and becomes in contact with the battery when the battery is put between the first and the second board retaining members is provided in an area of the flexible board arranged at one end side of the first board retaining member.

In the capsule medical apparatus, the casing includes a first casing member that has a cylindrical shape and a bottom and a second casing member that has a capsule shape and serves as a lid of the first casing member in engagement with the first casing member, the second board retaining member, the battery, and the first board retaining member are arranged in this order from a bottom side in an inside of the first casing member, and the second casing member covers the first board retaining member inserted to the cylindrical part and is fitted in the first casing member in a state where the battery segment is elastically compressed.

The capsule medical apparatus according to claim 1, further includes: an imaging lens; and a lens retaining frame that retains the imaging lens, wherein the first board retaining member includes two members which are divided in a direction perpendicular to a longitudinal direction of the casing, determines a position of the lens retaining frame in the longitudinal direction of the casing and the direction perpendicular to the longitudinal direction by lodging at least a part of the lens retaining frame therebetween, and determines a position of the first board in the direction perpendicular to the longitudinal direction of the casing by lodging the first board therebetween.

In the capsule medical apparatus, each of the first and the second board retaining members includes a position determining part that determines a position in a longitudinal direction and in a direction perpendicular to the longitudinal direction with respect to the casing in the state of being housed in the inside of the casing.

In the capsule medical apparatus, the battery is attached to an end part of the second board retaining member at an opposite side of the bottom side of the first casing member, a transmission antenna that performs a communication with an external device is formed on the second board, the second board is assembled to the second board retaining member so that a surface on which the transmission antenna is formed faces the bottom of the first casing member, a second battery segment that is provided with a tongue-like piece which protrudes to a side of the second board, is electrically connected to the second board, and has an elastic force is attached to a side of a connection surface between the battery and the second board retaining member, and the second board and the battery are electrically connected via the tongue-like piece while being away spatially.

In the capsule medical apparatus, the second board retaining member includes two members that are separated in a direction perpendicular to a longitudinal direction of the casing and determine a position in the longitudinal direction of the casing and in the direction perpendicular to the longitudinal direction by lodging the second board therebetween.

The capsule medical apparatus further includes: a second battery that is different from the battery and housed between the first board retaining member and the first battery, wherein a third battery segment that does not elastically deform is provided on an end surface of the second battery at the side of the first battery.

In the capsule medical apparatus, a groove part in which the flexible board is arranged is formed on an inner wall of the casing.

The capsule medical apparatus further includes: a third board on which a switch that switches on and off a power source in response to an external magnetic field is mounted, wherein the casing includes a part whose outer circumference has a cylindrical shape, a planar part is formed in a part of the outer circumference in the part having the cylindrical shape, and an arranging direction of the third board is determined depending on a position of the planar part.

In the capsule medical apparatus, a groove part in which the flexible board is arranged and whose position in a circumferential direction is different from the position of the planar part is formed on an inner wall of the casing.

In the capsule medical apparatus, the second casing member includes a hemispherical part formed of a transparent resin material and a cylindrical part coupled to the hemispherical part.

In the capsule medical apparatus, a parting line generated in molding is provided in a boundary part between the cylindrical part and the hemispherical part.

A method for manufacturing a capsule medical apparatus not forming part of the present invention includes: (a) making a first board retaining member retain a first board in the first and a second boards in at least one of which an imaging element is provided and which are connected by a flexible board; (b) making a second board retaining member retain the second board; and (c) housing the second board retaining member, a battery, and the first board retaining member in this order in an inside of a casing that is provided with first and second casing members and has a capsule shape.

The method for manufacturing a capsule medical apparatus further includes: attaching in an area of the flexible board arranged at one end side of the first board retaining member, before the process (a), a battery segment that elastically deforms and becomes in contact with the battery when housed at the process (c), wherein the process (c) includes covering the first board retaining member inserted to the cylindrical part by the second casing member and fitting the second casing member in the first casing member in a state where the battery segment is elastically compressed.

The method for manufacturing a capsule medical apparatus further includes: attaching, before the process (c), the battery in an end part of the second board retaining member at a side facing the first board retaining member when housed in the casing.

In the method for manufacturing a capsule medical apparatus, the process (c) includes inserting, between the battery and the first board retaining member, a second battery that is different from the battery and on one end surface of which a battery segment that does not elastically deform is attached by making the battery segment that does not electrically deform face a first, battery side.

### EFFECT OF THE INVENTION

According to the present invention, it is possible to assemble a capsule medical apparatus easily and to shorten a time required for the assembling since a battery is sandwiched between a first board retaining member retaining a first board and a second board retaining member retaining a second board, and the first board retaining member, the second retaining member, and the battery are housed in a casing in this state.

### BRIEF DESCRIPTION OF DRAWINGS

FIG. 1 is a cross sectional view of a configuration of a capsule medical apparatus according to an embodiment of the present invention;
FIGS. 2A and 2B are plane views of an external appearance of functional units provided in the capsule medical apparatus shown in FIG. 1;
FIG. 3 is a functional block diagram of a configuration of functional units provided in the capsule medical apparatus shown in FIG. 1;
FIG. 4A is a plane view of the dome part shown in FIG. 1 seen from an opening side;
FIG. 4B is a cross sectional view along the line A-A in FIG. 4A;
FIG. 5A is a plane view of the case part shown in FIG. 1 seen from an opening side;
FIG. 5B is a cross sectional view along the line B-B in FIG. 5A;
FIG. 6A is a cross sectional view along the line C-C in FIG. 5A;
FIG. 6B is a cross sectional view of a case where the case part is cut, as a cut surface, along the line D-D shown in FIG. 6A;
FIG. 7 is a perspective view of the first block part shown in FIG. 1;
FIG. 8 is a perspective view of a first spacer divided body constituting the spacer shown in FIG. 7;
FIG. 9 is a perspective view of a second spacer divided body constituting the spacer shown in FIG. 7;
FIG. 10 is a cross-sectional view along the line E-E in FIG. 7;
FIG. 11 is a perspective view of the third lens shown in FIG. 10;
FIG. 12A is a perspective view of the lens frame shown in FIG. 10 seen from the imaging element side;
FIG. 12B is a perspective view of the lens frame shown FIG. 10 seen from a side from which a light enters;
FIG. 13 is a flowchart of a method for assembling the objective lens unit shown in FIG. 10;
FIG. 14 is a plane view of the third lens and the lens frame the positions of which are adjusted;
FIG. 15 is a plane view of the negative electrode contact point spring shown in FIG. 1;
FIG. 16 is a cross sectional view along the line F-F in FIG. 15;
FIG. 17 is a flowchart of a method for assembling the first block part shown in FIG. 7;
FIG. 18 is a perspective view of an external appearance of the first battery lot shown in FIG. 1;
FIG. 19 is a perspective view of a structure of the positive electrode contact point member shown in FIG. 18;
FIG. 20 is an explanatory view of a method for manufacturing the first battery lot shown in FIG. 18;
FIG. 21 is a perspective view of an external appearance of the second battery lot shown in FIG. 1;
FIG. 22 is an explanatory view of a structure at a negative electrode surface side of a general disk-shaped battery;
FIG. 23 is a perspective view of a structure of the insulation sheet shown in FIG. 21;
FIG. 24 is an explanatory view of a method for manufacturing the second battery lot shown in FIG. 21;
FIG. 25 is a top view of an external appearance of the second block part shown in FIG. 1;
FIG. 26 is a cross sectional view along the line G-G in FIG. 25;
FIG. 27 shows a state where a first spacer divided body and a second spacer divided body are separated;
FIG. 28 shows a state where the first spacer divided body and the second spacer divided body are fitted with each other;
FIG. 29 is an enlarged perspective view of a positive electrode contact point member engagement part;
FIG. 30 is a cross sectional view of a case where the spacer is cut, as a cross sectional surface, along the line H-H in FIG. 26;
FIG. 31 is an enlarged perspective view of a positive electrode contact point member position regulating part;
FIGS. 32A to 32C are explanatory views of a method for installing a first electrode lot to the second block part;
FIG. 33 is a flowchart of a method for assembling the capsule medical apparatus shown in FIG. 1;
FIG. 34A shows a step of inserting the second block part and the first battery lot into the case part;
FIG. 34B shows a step of inserting the second battery lot into the case part;
FIG. 34C shows a step of inserting the first block part into the case part;
FIG. 34D shows a step of putting the dome part over the first block part;
FIG. 34E shows a completed capsule medical apparatus;
FIG. 35 is a flowchart of an operation of the capsule medical apparatus shown in FIG. 1;
FIG. 36 is a top view of a shape of an illumination board according to a first modification;
FIG. 37 is a top view of a shape of an illumination board according to a second modification;
FIG. 38 is a cross sectional view of an external form of an illumination board and a lens frame according to a third modification;
FIG. 39 is a perspective view of an external form of an illumination board and a lens frame according to a fourth modification;
FIG. 40 is a perspective view of a shape of a spacer and a board part on which a negative electrode contact point spring is mounted according to a fifth modification;
FIG. 41 is a partial cross sectional view illustrating a fold part of the flexible board;
FIG. 42 is a front view of an example of manufacturing a flexible board according to a sixth modification;
FIG. 43 is a cross sectional view of another example of manufacturing a flexible board according to the sixth modification;
FIG. 44 is a cross sectional view of still another example of manufacturing a flexible board according to the sixth modification; and
FIG. 45 is a top view of still another example of manufacturing a flexible board according to the sixth modification.

### BEST MODE FOR CARRYING OUT THE INVENTION

An exemplary embodiment of a capsule medical apparatus according to the present invention will be explained below with reference to the accompanying drawings. Here, the present invention is limited to the embodiment. Throughout the explanation of the drawings, a common part will be provided with a common reference symbol. It should be noted that the accompanying drawings are merely schematic and dimensional relations and ratio among parts may be different from the reality. Besides, there may be parts whose dimensional relations and the ratio are mutually different in the drawings.

### Embodiment

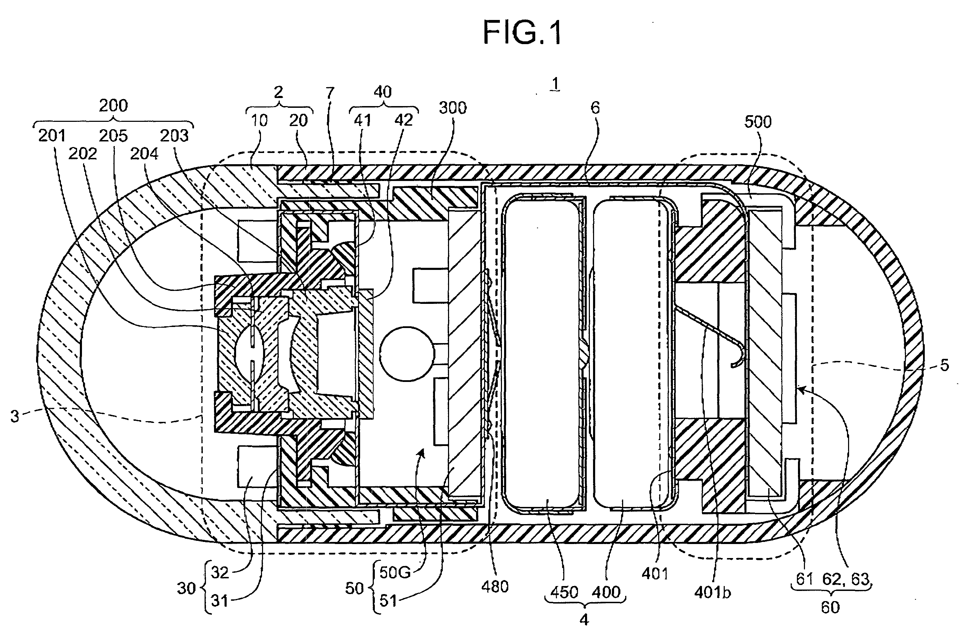

FIG. 1 is a cross sectional view of a configuration of a capsule medical apparatus according to an embodiment of the present invention. As shown in FIG. 1, a capsule medical apparatus 1 is provided with a capsule-shaped casing 2 constituted by a dome part 10 having a hemispherical shape and a case part 20 having a cylindrical part and a bottom; a first block part 3 in which functional units of various kinds are assembled in a spacer 300; a battery unit 4; and a second block part 5 in which functional units of various kinds are assembled in a spacer 500. The functional units assembled in the first block part 3 and the functional units assembled in the second block part 5 are electrically connected via a flexible board 6. The second block part 5, the battery unit 4, and the first block part 3 are housed in the case part 20 in this order and sealed within the casing 2 by the dome part 10 which serves as a lid in engagement with the case part 20.

The first block part 3 is provided with an illumination unit 30 that generates an illumination light for illuminating a subject, an objective lens unit 200 that condenses an incoming light from an outside via the dome part 10 to form an image on a light receiving part of an imaging element 42, an imaging unit 40 that performs a photoelectric conversion of a light having received via the objective lens unit 200 to generate an electrical signal indicating an image, and a control unit 50 that performs various controls such as a control of turning on and off a power of the capsule medical apparatus 1. Among these components, the objective lens unit 200 is designed so that an entrance pupil corresponds to a spherical center of the dome part 10. A negative electrode contact point spring 480 that allows securing an electrical conduction with the battery unit 4 is provided in the control unit 50.

The battery unit 4 is provided with a first battery lot 400 and a second battery lot 450 in each of which a battery, a contact point member, and the like are united.

The second block unit 5 is provided with a wireless communication unit 60 that receives an electrical signal generated in the imaging unit 40 and transmits a wireless signal after performing a superimposition thereon.

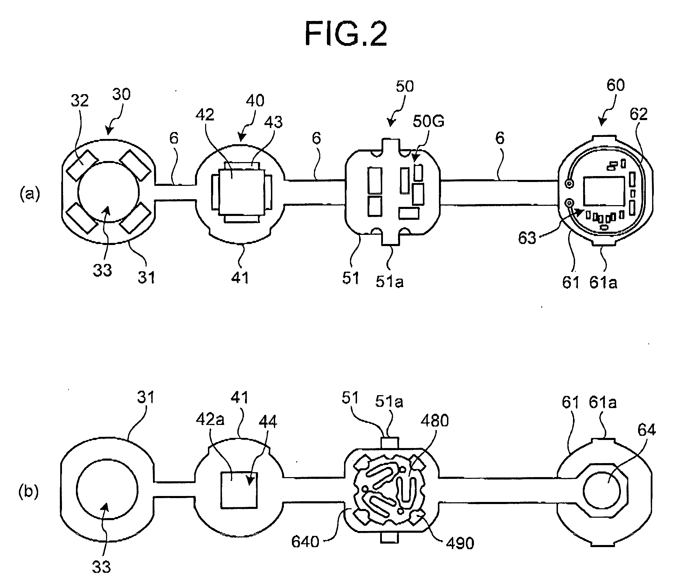

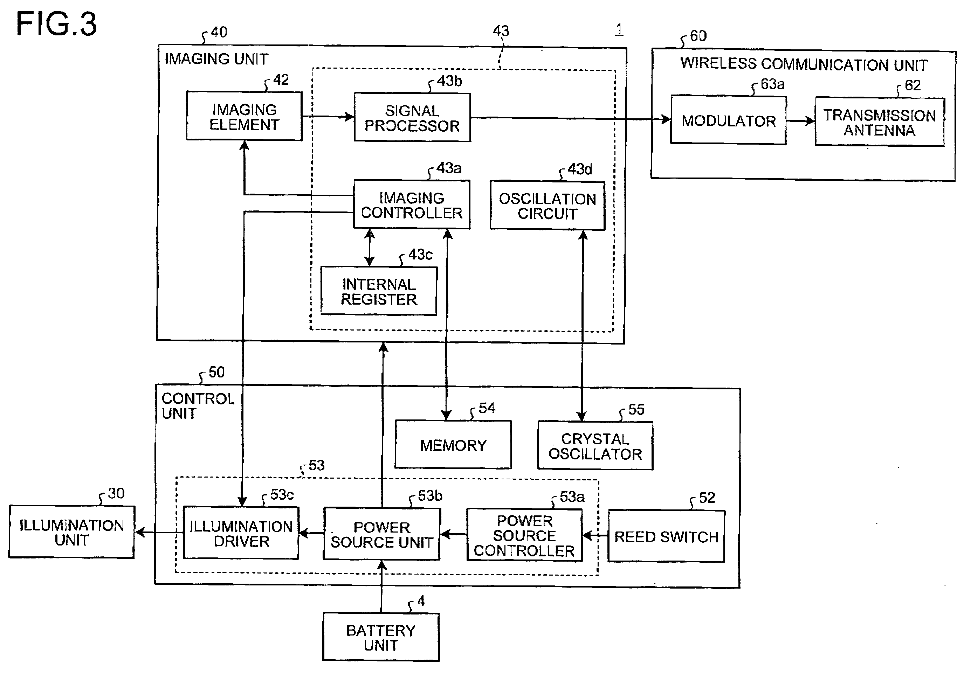

FIG. 2 are plane views schematically showing an external appearance of the illumination unit 30, the imaging unit 40, the control unit 50, and the wireless communication unit 60 which are connected via the flexible board 6. FIG. 2(a) shows a state where the illumination unit 30 is turned up and 2(b) shows a state where the illumination unit 30 is turned over upside down. FIG. 3 is a block diagram of a configuration of those functional units.

The illumination unit 30 is provided with an illumination board 31 which is flexible and integrally formed with the flexible board 6 and a plurality of illumination elements 32 that are mounted on the illumination board 31. An opening 33 having a circular shape is formed nearly at a center part of the illumination board 31 and the illumination elements 32 are arranged around the opening 33. The illumination elements 32 are, for example, LEDs which generate a white light. In the embodiment, four illumination elements 32 are provided around the opening 33 at even intervals. The illumination elements 32 are connected in series to be connected to an illumination driving circuit which will be explained later.

A position of the illumination unit 30 in the inside of the casing 2 is determined by inserting the objective lens unit 200 into the opening 33 of the illumination board 31.

The imaging unit 40 is provided with an imaging board 41 which is flexible and integrally formed with the flexible board 6, an imaging element 42 like a CMOS and the like which is mounted on the imaging board 41 by the flip chip method, and a circuit unit 43 that makes the imaging element 42 execute an imaging operation. An opening 44 having a rectangular shape is formed nearly at a center part of the imaging board 41. The imaging element 42 is provided so that a light-receiving surface 42a is oriented to a side of the imaging board 41 and a periphery of the light-receiving surface 42a comes to be in abutting contact with the periphery of the opening 44. This configuration allows the light-receiving surface 42a to be exposed from the opening 44 as shown in FIG. 2(b). The imaging element 42 receives a light having passed through the opening 44 and performs a photoelectric conversion to generate an electrical signal indicating an image corresponding to the subject. The circuit unit 43 is provided with an imaging controller 43a that controls an imaging operation by the imaging element 42 and controls an operation of an illumination driver 53c (to be explained later) in synchronization with the imaging operation, a signal processor 43b that executes a predetermined signal process on the electrical signal generated by the imaging element 42 to perform a conversion into an image signal, an internal register 43c that stores information concerning the capsule medical apparatus 1 (ID information and the like), and an oscillation circuit 43d that generates a clock signal based on an oscillation generated by a crystal oscillator 55 which will be explained later. The imaging element 42 and the circuit unit 43 may be provided in the same IC chip or provided in separated IC chips.

The control unit 50 is provided with a control board 51 formed by a rigid board and an electronic part group 50G including a reed switch 52, a power source IC 53, a memory 54, a crystal oscillator 55, and the like mounted on the control board 51. The control board 51 is electrically connected to the flexible board 6 by soldering. In an area different from a part from which the flexible board 6 extends within an edge area of the control board 51, a lug part 51a used for a positional adjustment with the spacer 300 is formed in such a shape as to protrude outward from the flexible board 6. The reed switch 52 performs a switching operation in response to an external magnetic field. The power source IC 53 is provided with a power source controller 53a that performs a control of activating and stopping the power source in response to the switching operation of the reed switch 52, a power source unit 53b that supplies a power to the illumination unit 30 and the imaging unit 40 under the control of the power source controller 53a, and an illumination driver 53c that drives the illumination unit 30. The memory 54 is, for example, an EEPROM and stores operation setting information and the like. In a board part 640 formed integrally with the flexible board 6 at a rear side of the control board 51, the negative electrode contact point spring 480 is mounted by using a solder 490.

The wireless communication unit 60 is provided with a board 61 which is used for a wireless communication and formed by a rigid board (hereinafter referred to as "wireless board"), an antenna 62 which is used for a wireless signal transmission (hereinafter referred to as "transmission antenna") and formed on the wireless board 61, and an electronic part 63 which is used for a wireless communication and mounted on the wireless board 61. The wireless board 61 is electrically connected to the flexible board 6 by the solder 490. In an area different from a part from which the flexible board 6 extends within an edge area of the wireless board 61, a lug part 61a used for a positional adjustment with the spacer 500 is formed. In the embodiment, the transmission antenna 62 is realized by forming an antenna on the wireless board 61 via patterning to suppress a variation in manufacturing. The electronic part 63 includes, for example, an element constituting a modulator 63a that modulates an image signal output from the imaging unit 40 and the like. On a flexible board surface at a rear side of the wireless board 61, a pad 64 that is electrically connected to the first battery lot 400 is provided.

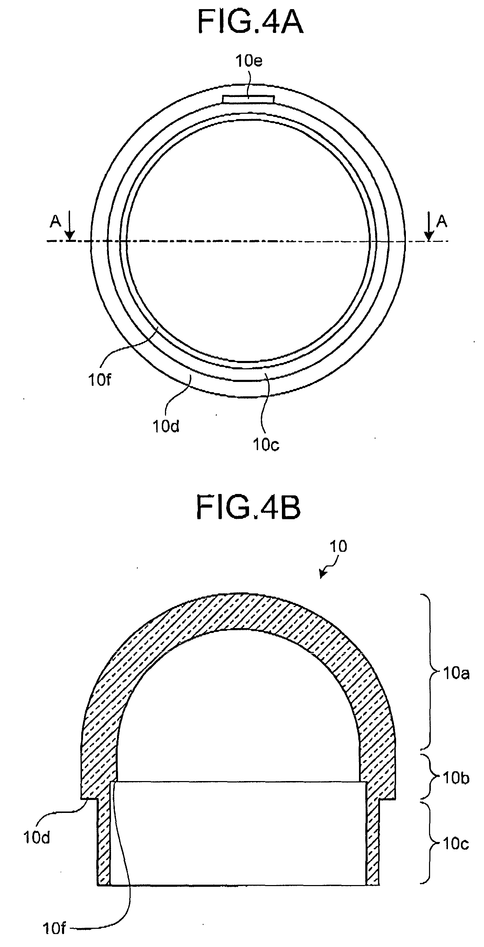

Next, the casing 2 will be explained in detail. FIG. 4A is a plane view of the dome part 10 seen from the opening side. FIG. 4B is a cross sectional view along the line A-A in FIG. 4A.

The dome part 10 is provided with a dome hemispherical part 10a which has a hemispherical shape, a dome grasping part 10b which has a cylindrical shape and has the same outer diameter as the dome hemispherical part 10a, and a dome cylindrical part 10c which is provided with a cutout so that its outer diameter becomes smaller than that of the dome grasping part 10b and fitted with the case part 20. At a boundary between the dome hemispherical part 10a and the dome grasping part 10b, a parting line which is generated in molding may be arranged. Such a parting line enables the boundary between the dome hemispherical part 10a and the dome grasping part 10b to be visually recognized easily.

The dome hemispherical part 10a is a part which becomes one end part in a longitudinal direction of the capsule medical apparatus 1. A front surface, in an area included in a range of an optical field of view of the imaging unit 40, of the dome hemispherical part 10a is mirror-finished.

The dome grasping part 10b is provided so that the dome hemispherical part 10a is grasped without touching the mirror-finished part in the assembling and the like.

An outer diameter of the dome cylindrical part 10c is nearly equal to an inner diameter of a case fitting part 20c which will be explained later. Besides, an edge face 10d of the dome grasping part 10b is made to abut on an edge face of the case part 20 (a case edge part 20g) when the dome cylindrical part 10c is fitted with the case part 20. By providing such an edge face 10d, it is possible to perform an accurate positioning between the dome part 10 and the case part 20 in the longitudinal direction.

According to the invention, as shown in FIG. 4A, a rotation regulating part 10e which protrudes outward is formed on an outer circumferential surface of the dome cylindrical part 10c for regulating a rotation of the dome cylindrical part 10c with respect to the case part 20.

In addition, an inner wall rib 10f which allows positioning when the first block part 3 and the like are assembled to the inside of the casing 2 is formed at an inner circumference side of the dome part 10.

The dome part 10 has transparency with respect to an illumination light such as a visual light and the like radiated by the illumination unit 30 and is formed, via an injection molding, by using materials having biocompatibility (resin materials such as polycarbonate, acrylic, and cycloolefinpolymer, for example).

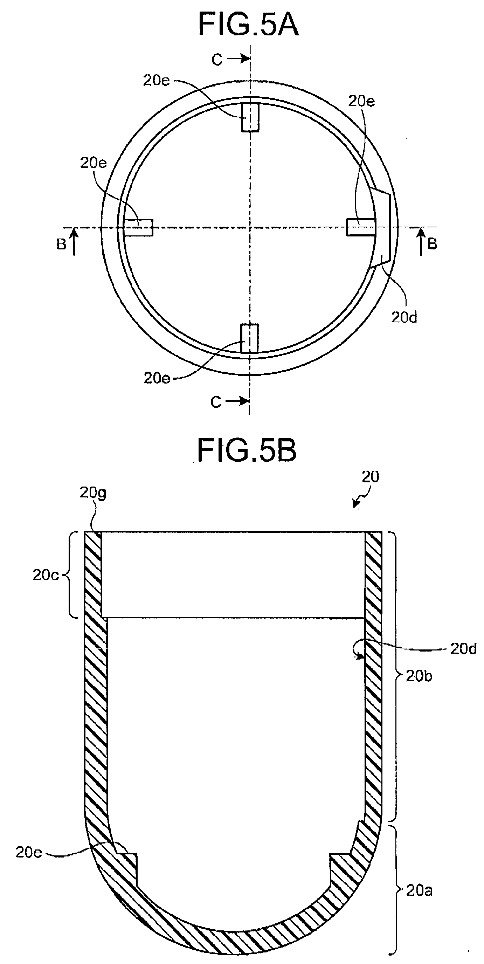

FIG. 5A is a plane view of the case part 20 seen from the opening side. FIG. 5B is a cross sectional view along the line B-B in FIG. 5A.

The case part 20 is provided with a case hemispherical part 20a which has a hemispherical shape and a case cylindrical part 20b whose outer diameter is the same as that of the case semispherical part 20a and which has a cylindrical shape. The case hemispherical part 20a is a part which becomes the other end part in the longitudinal direction of the capsule medical apparatus 1.

At an end part at the opening side of the case cylindrical part 20b, the case fitting part 20c with which the dome cylindrical part 10c is fitted is provided. An inner diameter of the case fitting part 20c is larger than an inner diameter of the rest part of the case cylindrical part 20b. By making the case fitting part 20c thinner than the rest part, it becomes possible to make a total thickness of the dome cylindrical part 10c and the case fitting part 20c comparable to the thickness of the rest part of the case cylindrical part 20b and to secure a sufficient inner space in a region of the case fitting part 20c.

A case groove part 20d is formed on an inner wall of the case part 20. The case groove part 20d is provided to allow securing a space where the flexible board 6 is arranged when the first block part 3 and the like are housed in the inside of the casing 2 and, according to the invention, also allow an engagement of the rotation regulating part 10e provided in the dome cylindrical part 10c in fitting the dome part 10.

According to the invention, as shown in FIG. 5A, a cross section perpendicular to the longitudinal direction of the case groove part 20d has a nearly trapezoidal shape in which a width at a bottom part side (outer circumference side when seen from the opening side) of the groove is narrower. This shape is adopted to suppress a sharp change in thickness in the injection molding of the case part 20 and to prevent detracting moldability.

At a plurality of locations at the inner side of the case hemispherical part 20a, a case inner wall rib 20e which protrudes toward an inner circumference side is formed for positioning when the second block part 5 and the like are housed in the inside of the casing 2. While the case inner wall rib 20e is provided at four locations in FIG. 5A, the number and the size of the case inner wall rib 20e are not limited thereto. For example, only one ringshaped case inner wall rib which continues over an entirety of the inner circumference of the case part 20 may be formed.

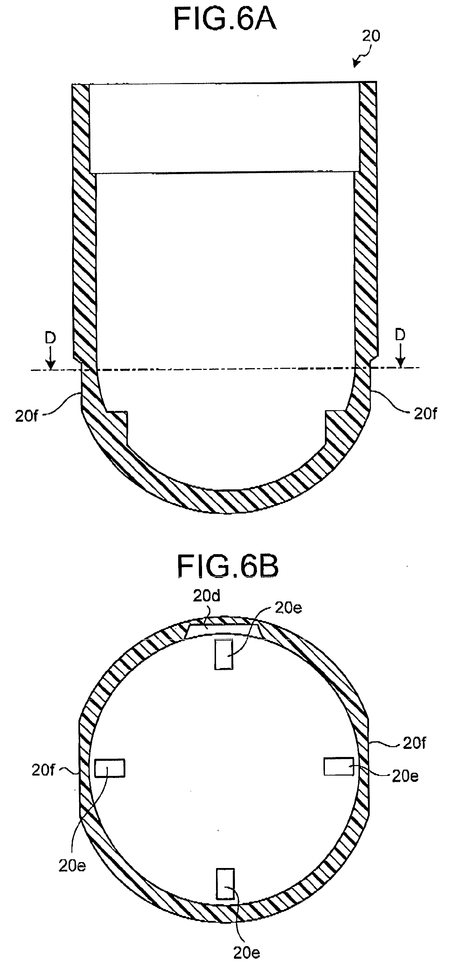

FIG. 6A is a cross sectional view along the line C-C in FIG. 5A and FIG. 6B is a cross sectional view of a case where the case part 20 is cut, as a cut surface, along the line D-D shown in FIG. 6A. As shown in FIGS. 6A and 6B, a planar part 20f (so-called "D cut") obtained by cutting a part of the outer circumference in a planar fashion is formed at the outer circumference side of the case cylindrical part 20b for determining an orientation of the casing 2. The planar part 20f is provided in a region of a part in the longitudinal direction of the case cylindrical part 20b (end part at the side of the case hemispherical part 20a, for example). While the position of the planar part 20f in the circumferential direction is not specifically limited, it is preferable that the position is displaced from the position of the case groove part 20d for securing a certain thickness of the case cylindrical part 20b. In the embodiment, the planar part 20f is provided at a position displaced by 90 degrees in the circumferential direction with respect to the case groove part 20d. It becomes possible to determine the orientation of the embedded components from the external appearance of the casing 2 by determining in advance the orientation of the components to be housed in the casing 2 with respect to the planar part 20f. While the planar part 20f is provided at two locations facing with each other in FIGS. 6A and 6B, the planar part 20f may be provided at one location.

The case part 20 may be opaque and colored and is formed, via an injection molding, by using materials having biocompatibility (resin materials such as polysulphone and polycarbonate, for example).

The casing 2 is sealed in a watertight manner by an adhesive agent 7 arranged between the outer circumference side of the dome cylindrical part 10c and the inner circumference side of the case fitting part 20c (see FIG. 1).

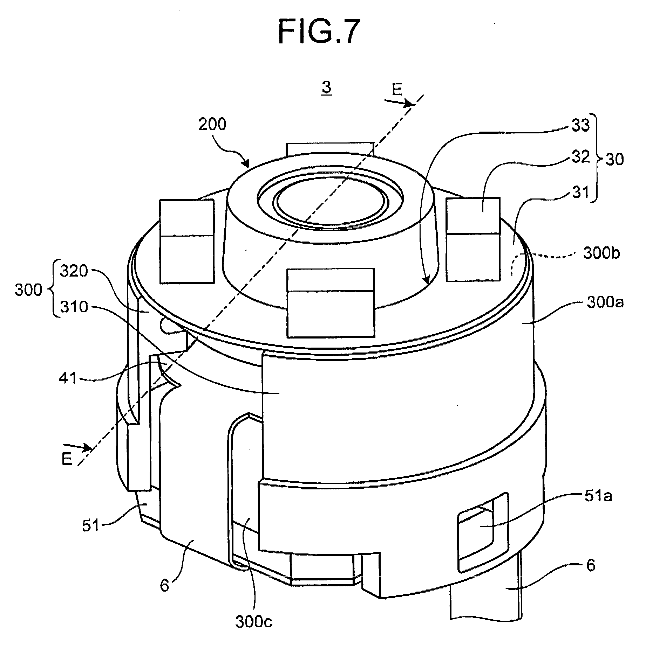

Next, the first block part 3 will be explained in detail. FIG. 7 is a perspective view of an external appearance of the first block part 3. An upper side in FIG. 7 is where the dome part 10 is arranged.

As shown in FIG. 7, the spacer 300 has a cylindrical contour as a whole and is constituted by a pair of divided bodies (a first spacer divided body 310 and a second spacer divided body 320) which are separated by a plane passing through a central axis of the cylindrical shape. The first spacer divided body 310 and the second spacer divided body 320 retain, by lodging therebetween, the objective lens unit 200, the illumination unit 30, the imaging unit 40, and the control unit 50.

A dome fitting part 300a is a part which is fitted with the inner wall of the dome cylindrical part 10c when retained by the dome part 10. An outer diameter of the dome fitting part 300a is nearly equal to the inner diameter of the dome cylindrical part 10c and determined so that an upper end part 300b of the spacer 300 is inserted in a manner of being allowed to directly contacting the inner wall rib 10f of the dome part 10.

The upper end part 300b of the spacer 300 serves as a receiving face of the illumination board 31. The illumination board 31 is arranged by inserting a protruding part of the objective lens unit 200 to the opening 33 from the upper end part 300b.

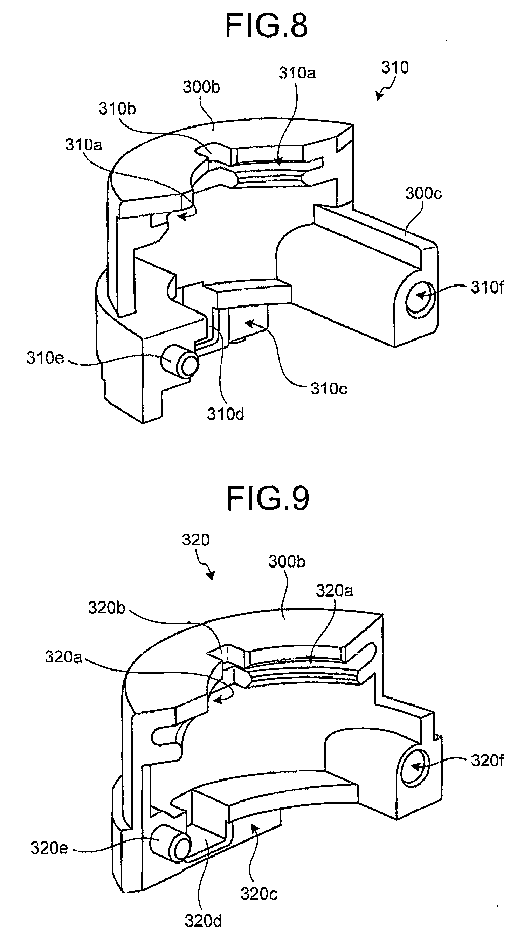

FIG. 8 is a perspective view of the first spacer divided body 310. In the first spacer divided body 310, a lens frame engagement part 310a that supports the objective lens unit 200 from the lateral direction is formed at two locations on the arc. A cross section of the lens frame engagement part 310a has an orthogonally inclined U-shape. Between these lens frame engagement parts 310a, a lens frame rotation regulating part 310b that regulates a positional displacement in the rotating direction of the objective lens unit 200 is provided.

Besides, a board engagement part 310c which laterally lodges the control board 51 is formed in the first spacer divided body 310. In this board engagement part 310c, a board rotation regulating part 310d that regulates a positional displacement in the rotating direction of the control board 51 in engagement with the lug part 51a of the control board 51 is provided.

A flexible board receiver (hereinafter referred to as "board receiver") 300c is formed in an outer circumferential part of the first spacer divided body 310. The board receiver 300c is a part which allows the flexible board 6 extending to the side of the control board 51 from the imaging board 41 to be arranged by letting it get out of the spacer 300 once without lodging it between the first spacer divided body 310 and the second spacer divided body 320. By letting the flexible board 6 get out of the spacer 300 in this manner, a folding amount of the flexible board 6 is made small and thereby damage on the flexible board 6 is reduced. A round chamfering is made along an edge line, where the flexible board 6 contacts, of the board receiver 300c. It is only necessary to provide the board receiver 300c in one of the first spacer divided body 310 and the second spacer divided body 320.

The first spacer divided body 310 is provided with a boss 310e and a fitting hole 310f as a fitting part for being united with the second spacer divided body 320.

FIG. 9 is a perspective view of the second spacer divided body 320. In the second spacer divided body 320, a lens frame engagement part 320a that supports the objective lens unit 200 from the lateral direction is formed at two locations on the arc. A cross section of the lens frame engagement part 320a has an orthogonally inclined U-shape. Between these lens frame engagement parts 320a, a lens frame rotation regulating part 320b that regulates a positional displacement in the rotating direction of the objective lens unit 200 is provided.

Besides, a board engagement part 320c which laterally lodges the control board 51 is formed in the second spacer divided body 320. In this board engagement part 320c, a board rotation regulating part 320d that regulates a positional displacement in the rotating direction of the control board 51 in engagement with the lug part 51a of the control board 51 is provided.

The second spacer divided body 320 is provided with a fitting hole 320f formed at a position facing the boss 310e of the first spacer divided body 310 and a boss 320e formed at a position facing the fitting hole 310f.

The first spacer divided body 310 and the second spacer divided body 320 are formed via an injection molding using resin materials such as polycarbonate, acrylonitrile-butadiene-styrene, polyoxymethylene (POM), and modified polyphenylene ether (PPE; modified PPO), for example. Especially, since being light in weight and of an adequate mechanical strength compared to other resins, the modified PPO is advantageous in that a crack hardly occurs in the assembled state and the like.

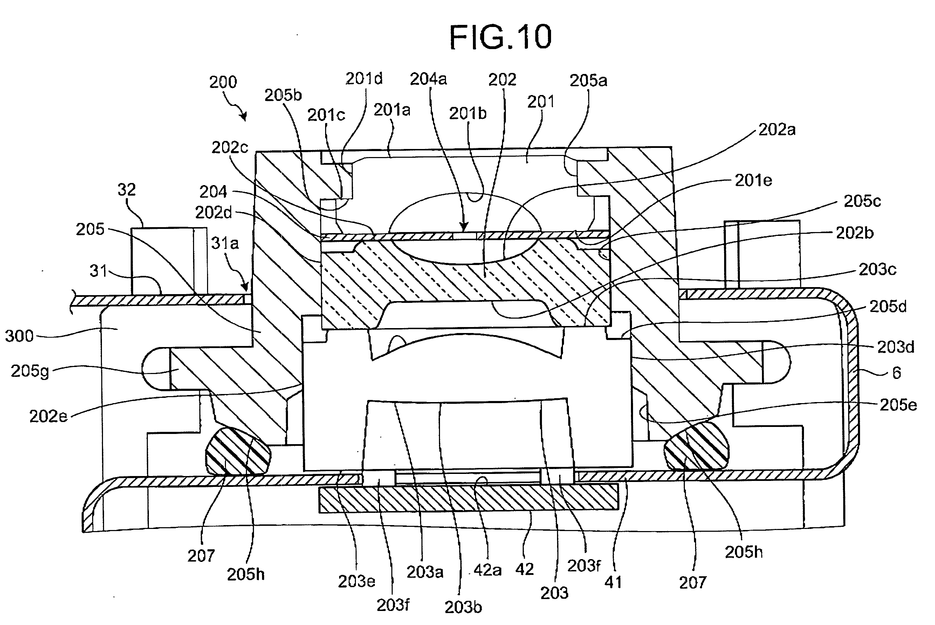

Next, the objective lens unit 200 will be explained in detail. FIG. 10 shows a part of the cross section along the line E-E in FIG. 7.

The objective lens unit 200 is provided with first to third lenses 201 to 203, an aperture 204, and a lens frame 205 that positions and retains these optical parts.

The objective lens unit 200 is positioned with respect to the light-receiving surface 42a of the imaging element 42 by an imaging element abutting part 203f provided in the third lens 203. The objective lens unit 200 and the imaging board 41 are fixed to each other by an adhesive agent 207. In addition, an optical system in the objective lens unit 200 is sealed by this adhesive agent 207.

A spacer fitting part 205g that allows the lens frame engagement parts 310a and 320a of the spacer 300 to lodge the objective lens unit 200 is provided in the lens frame 205 of the objective lens unit 200.

The first to the third lenses 201 to 203 are transparent lenses formed via the injection molding using resins such as cycloolefinpolymer (COP), polycarbonate, and acrylic, for example and arranged so that respective optical axes get together mutually.

As shown in FIG. 10, the first lens 201 is a concave lens having a first lens surface 201a and a second lens surface 201b which face to each other and arranged by putting the first lens surface 201a to a direction from which a light enters. A lens frame abutting part 201c is provided in a marginal part at the side of the first lens surface 201a of the first lens 201 (outer side of the lens surface) in a manner of being perpendicular to the optical axis. Besides, a part of the side surface of the outer circumference of the first lens 201 serves as a lens frame fitting part 201d formed coaxially with the optical axis in a cylindrical shape. An aperture receiver 201e to be in direct contact with the aperture 204 is provided in a marginal part of an end face at the side of the second lens surface 201b of the first lens 201 in a manner of being perpendicular to the optical axis.

The second lens 202 is a concave lens having a first lens surface 202a and a second lens surface 202b which face to each other and arranged by putting the first lens surface 202a at the side of the first lens 201. An aperture receiving surface 202c to be in direct contact with the aperture 204 is provided in a marginal part of an end face at the side of the first lens surface 202a of the second lens 202 in a manner of being perpendicular to the optical axis and projecting from the first lens surface 202a. Besides, a part of the side surface of the outer circumference of the second lens 202 serves as a lens frame fitting part 202d formed coaxially with the optical axis in a cylindrical shape. A diameter of the lens frame fitting part 202d is configured to be larger than a diameter of the lens frame fitting part 201d. A lens receiver 202e to be in direct contact with the third lens 203 is provided in a marginal part of an end face at the side of the second lens surface 202b of the second lens 202 in a manner of being perpendicular to the optical axis.

The third lens 203 is a convex lens having a first lens surface 203a and a second lens surface 203b which face to each other and arranged by putting the first lens surface 203a at the side of the second lens 202. A lens abutting part 203c to be in direct contact with the second lens 202 is provided in a marginal part of an end face at the side of the first lens surface 203a of the third lens 203 in a manner of being perpendicular to the optical axis and projecting from the first lens surface 203a. Besides, a part of the side surface of the outer circumference of the third lens 203 serves as a lens frame fitting part 203d formed coaxially with the optical axis in a cylindrical shape. A diameter of the lens frame fitting part 203d is configured to be larger than the diameter of the lens frame fitting part 202d.

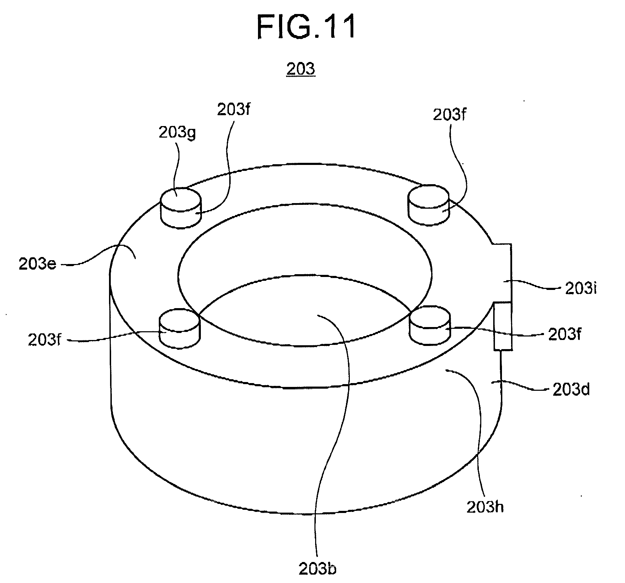

FIG. 11 is a perspective view of the third lens 203 seen from the side of the second lens surface 203b. In a marginal part of a bottom face 203e at the side of the second lens surface 203b, a plurality of imaging element abutting parts 203f are provided. It is only necessary to determine the number and the arrangement of the imaging element abutting parts 203f depending on a size and a shape of the opening 44 of the imaging board 41 and the light-receiving surface 42a of the imaging element 42, and the imaging element abutting part 203f is provided at four locations corresponding to respective inner side edge parts of four vertexes of the opening 44 in the embodiment.

The imaging element abutting parts 203f have a columnar shape and are formed so that four abutting surfaces 203g to be in direct contact with the imaging element 42 are included on the same plane perpendicular to the optical axis.

In a side surface area 203h at the side of the bottom face 203e of the third lens 203 (an area which is not in contact with the lens frame fitting part 203d), a positioning land mark part 203i for determining the orientation of the imaging element abutting parts 203f is formed. The positioning land mark part 203i can be substituted by a residue of a cut gate generated in the injection molding of the third lens 203.

Referring again to FIG. 10, the aperture 204 is arranged for determining the brightness and the focal depth of the optical system. For the aperture 204, a black color metal aperture generated by forming metal, for example, phosphor bronze and the like, into a thin plate and processing it via a punch-out process, an etching, and the like, a black color resin aperture generated by performing a punch-out process of a resin sheet of polyester and the like, and the like are used. The aperture 204 has nearly the same outer diameter as the lens frame fitting part 202d of the second lens 202 and has a donut-like shape in which a circular opening 204a is formed in its center coaxially with the outer circumference.

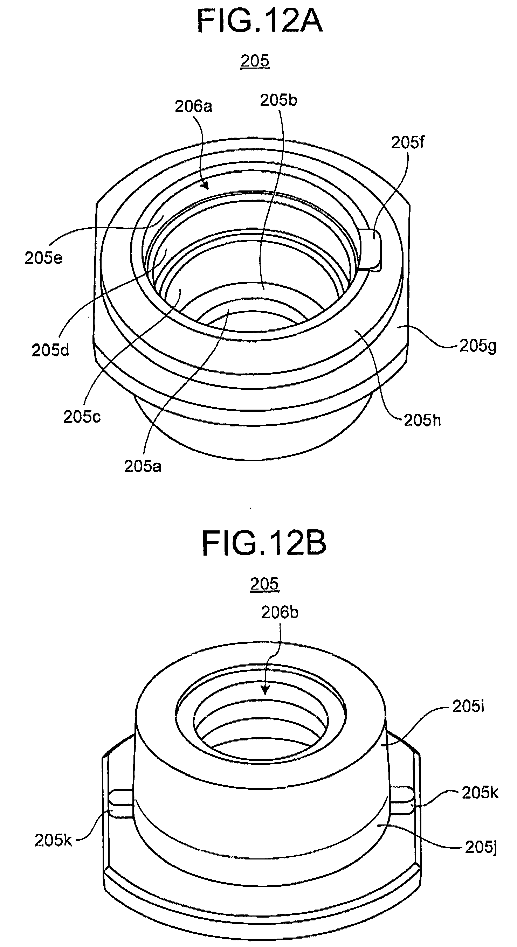

FIG. 12A is a perspective view of the lens frame 205 seen from the imaging element side and FIG. 12B is a perspective view of the same seen from the side from which a light enters.

The lens frame 205 is provided to bring respective optical axes of the first to the third lenses 201 to 203 and the aperture 204 in line and to retain them with intervals of respective lens surfaces regulated. The lens frame 205 is manufactured by an injection molding using a resin such as polycarbonate (PC), for example or a cutting process using a metal such as a stainless steel and a brass. The lens frame 205 is preferably a black color for the sake of a light shielding.

The lens frame 205 is a frame body having a cylindrical structure whose both ends are open and is provided with an insertion opening 206a to which the first to the third lenses 201 to 203 and the aperture 204 are inserted in the assembling and an incident light opening 206b from which the illumination light enters in the imaging.

A lens fitting part 205a whose diameter is nearly equal to that of the lens frame fitting part 201d of the first lens 201 and which is provided coaxially with the lens frame fitting part 201d of the first lens 201 is formed on an inner wall in the vicinity of an end part at the side of the incident light opening 206b of the lens frame 205. Besides, a lens receiver 205b to be in direct contact with the lens frame abutting part 201c is formed at a side of the periphery of the lens fitting part 205a in a manner of being perpendicular to the optical axis.

An inner wall side surface at an upper side of the lens receiver 205b in an aspect shown in FIG. 12A is a lens fitting part 205c whose diameter is nearly equal to that of the lens frame fitting part 202d of the second lens 202 and which is provided coaxially with the lens frame fitting part 202d of the second lens 202. Besides, an inner wall side surface at an upper side of the lens fitting part 205c is a lens fitting part 205d whose diameter is nearly equal to that of the lens frame fitting part 203d of the third lens 203 and which is provided coaxially with the lens frame fitting part 203d of the third lens 203. Moreover, a gap forming part 205e whose diameter is larger than that of the lens fitting part 205d is provided at an upper side of the lens fitting part 205d. This gap forming part 205e forms a gap which enables the positioning land mark part 203i (FIG. 11) to be housed between the side surface 203h of the third lens 203 and the lens frame 205 when the third lens 203 is fitted.

A positioning land mark part 205f is formed at an outside of the opening of the end face at the side of the insertion opening 206a of the lens frame 205. In addition, a spacer fitting part 205g that enables the spacer 300 to retain the lens frame 205 is formed in the vicinity of the end part at the side of the insertion opening 206a.

A slope part 205h that slopes outward to the side of the spacer fitting part 205g is provided in the periphery of the opening of the end face at the side of the insertion opening 206a. This slope part 205h allows securing a space in which the adhesive agent 207 is arranged between the lens frame 205 and the imaging board 41.

As shown in FIG. 12B, the lens frame 205 is provided with an illumination board installation part 205i which has a tapered shape of becoming widened from the side of the incident light opening 206b to the insertion opening 206a and a cylindrical part 205j that has the same diameter as a lower end of the illumination board installation part 205i. The illumination board installation part 205i is a part to be inserted to an opening 31a formed in the illumination board 31 and an easy insertion thereto is realized by making the contour slightly have a tapered shape. The diameter of the lower end of the illumination board installation part 205i is configured to be nearly equal to the diameter of the opening 31a for the sake of the positioning of the illumination board 31 with respect to the lens frame 205. On the other hand, the cylindrical part 205j is a part to be fitted with the spacer 300.

On the side surface of the cylindrical part 205j on the spacer fitting part 205g, spacer rotation regulating parts 205k that regulate, in engagement with the lens frame rotation regulating parts 310b and 320b of the spacer 300, a positional displacement of the lens frame 205 with respect to the spacer 300 are provided.

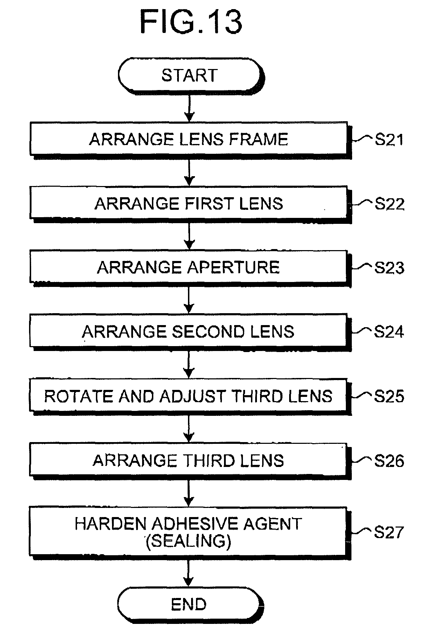

Next, a method for assembling the objective lens unit 200 will be explained with reference to FIG. 13.

First at step S21, the lens frame 205 is placed at a stable site so that the insertion opening 206a is turned up (the aspect shown in FIG. 12A).

At subsequent step S22, the first lens 201 is inserted through the insertion opening 206a in a state where the first lens surface 201a is casted down, so that the lens frame fitting part 201d is fitted with the lens fitting part 205a and the lens frame abutting part 201c is brought to a state of being in direct contact with the lens receiver 205b. Thus, the positioning of the first lens 201 in the radial direction and the axis direction is completed.

At subsequent step S23, the aperture 204 is inserted through the insertion opening 206a, so that the outer circumference is fitted with the lens fitting part 205a and one end plane is brought to a state of being in direct contact with the aperture receiver 201e. Thus, the positioning of the aperture 204 in the circumferential direction and the axial direction is completed.

At subsequent step S24, the second lens 202 is inserted through the insertion opening 206a in a state where the first lens surface 202a is casted down, so that the lens frame fitting part 202d is fitted with the lens fitting part 205a and the aperture receiving surface 202c is brought to a state of being in direct contact with the aperture 204. Thus, the positioning of the second lens 202 in the circumferential direction and the axis direction is completed.

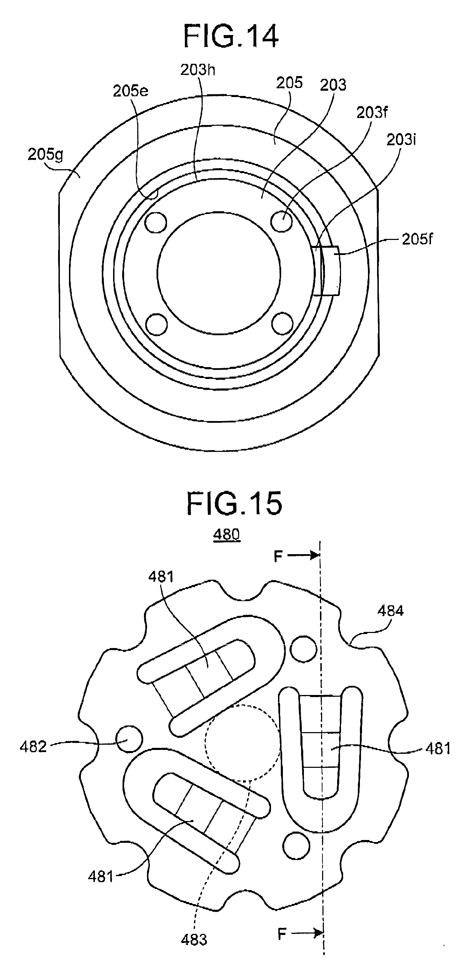

At subsequent step S25, after a thermoset, an ultraviolet cure, or other adhesive agent is applied on an entire circumference of the lens frame fitting part 203d of the third lens 203, the third lens 203 is rotated and adjusted by being viewed by only eyes or under a microscope so that the positioning land mark part 203i of the third lens 203 meets the positioning land mark part 205f of the lens frame 205 as shown in FIG. 14. By this adjustment, the position of the four imaging element abutting parts 203f with respect to the lens frame 205 is determined.

Furthermore, at step S26, the third lens 203 is inserted through the insertion opening 206a in a state where the first lens surface 203a is casted down. Then, the lens frame fitting part 203d is fitted with the lens fitting part 205d and the lens abutting part 203c is brought to a state of being in direct contact with the lens receiver 202e of the second lens 202. Thus, the positioning of the third lens 203 in the circumferential direction and the axis direction is completed.

At step S27, the inside of the lens frame 205 is sealed by making the adhesive agent applied on the lens frame fitting part 203d of the third lens 203 hardened. Thus, the objective lens unit 200 is completed.

Next, the negative electrode contact point spring 480 arranged in the board part 640 having flexibility at the rear side of the control board 51 will be explained with reference to FIG. 15. FIG. 15 is a plane view of the negative electrode contact point spring 480.

The negative electrode contact point spring 480 is elastically compressed in the state of being housed in the inside of the casing 2, electrically connects the control board 51 and the second battery lot 450, absorbs a tolerance in the inside of the casing 2, and biases the components embedded in the casing 2 to both directions from the negative electrode contract point spring 480 as a border.

The negative electrode contact point spring 480 is provided with three spring parts 481 that keeps a conduction while biasing the facing second battery lot 450 (see FIG. 1), three protruding parts 482, a center plane part 483, and a cutout part 484 provided in the outer circumferential part.

The spring parts 481 are arranged around the center plane part 483 at respective positions which have rotational symmetries through 120 degrees centering around a center of the center plane part 483. By supporting a battery 453 at the three points having rotational symmetries centering around the center of the center plane part 483 in this manner, it is possible to arrange the battery 453 in a stable state on the negative electrode contact point spring 480.

A load (imposed load) on the spring part 481 is set so that a contact resistance against the battery 453 is equal to or less than 500 mΩ. Besides, a height (displacement) of the spring part 481 is set to absorb a variation in length in the axial direction of the components embedded in the casing 2. The imposed load is set so that a force falling within a range of the contact resistance explained above can be generated in the range of the variation in the axial direction.

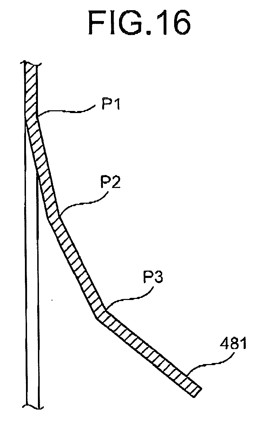

FIG. 16 is a cross sectional view along the line F-F in FIG. 15. As shown in FIG. 16, the spring part 481 is bent at three positions P1, P2, and P3. This configuration allows gaining a total amount of the displacement of the spring part 481. In addition, this configuration allows decentralizing, in position, a stress to be generated when the spring part 481 is bent and thereby suppressing degradation of spring characteristics due to plastic deformation.

The protruding parts 482 are arranged, by being displaced from the positions of the spring parts 481, in a manner of having rotational symmetries through 120 degrees with respect to the center of the center plane part 483. The protruding part 482 is provided in a manner of protruding in the same direction as the bending direction of the spring part 481 (on the near side of the paper plane in FIG. 15). This configuration allows preventing the spring part 481 from being bent deeply beyond a predetermined height and thereby preventing the degradation of spring characteristics due to the plastic deformation more surely.

The center plane part 483 is used as a spring suction face in an automatic implementation of the negative electrode contact point spring 480. A size of the center plane part 483 is preferably equal to or more than 4.5 mm in diameter approximately, for example. The size is determined by a shape of a suction part capable of sucking the negative electrode contact point spring 480.

The cutout part 484 is provided at predetermined intervals in the outer circumference of the negative electrode contact point spring 480. The cutout part 484 has a shape of R0.25 mm, for example. The shape is determined depending on a solder land shape in mounting the negative electrode contact point spring 480.

The negative electrode contact point spring 480 is formed by a press molding process using metal thin plate having spring characteristics such as a stainless steel
(SUS304CSP, for example) and a phosphor bronze (C5210P, for example), and then manufactured via a base process using Ni-P plating approximately as thick as 5 µm and a surface-finishing process using Au-Co plating approximately as thick as 0.5 µm, for example. Besides, the negative electrode contact point spring 480 is fixed on and has conduction with a part of the flexible board 6 at the rear side of the control board 51 by soldering at four points at intervals of 90 degrees, for example in the cutout parts 484 provided in the outer circumferential part.

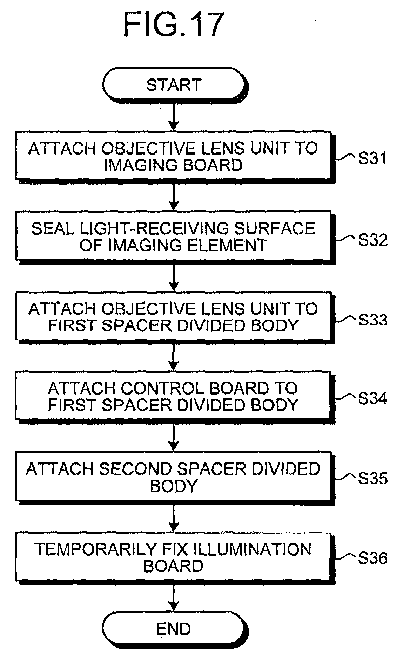

Next, a method for assembling the first block part 3 will be explained with reference to FIG. 17.

First at step S31, the imaging board 41 is attached to the objective lens unit 200. Specifically, the imaging element abutting part 203f is made to abut on the light-receiving surface 42a of the imaging element 42 via the opening 44 of the imaging board 41 while referring to the positioning land mark part 205f (FIG. 14) of the lens frame 205. After that, the imaging board 41 and the objective lens unit 200 are temporarily fixed by using an adhesive agent of a UV cure type and the like.

At step S32, the adhesive agent 207 of the UV cure type and the like is arranged between the slope part 205h of the lens frame 205 and the imaging board 41 and hardened. Thus, a space including the light-receiving surface 42a of the imaging element 42 is sealed, thereby suppressing a degradation of optical characteristics due to an invasion of dust, humidity, and the like.

At step S33, the objective lens unit 200 is attached to the first spacer divided body 310. Specifically, the spacer fitting part 205g is made to engage with the lens frame engagement part 310a from the lateral direction in a state where the spacer rotation regulating part 205k of the lens frame 205 is made to engage with the lens frame rotation regulating part 310b of the first spacer divided body 310. On this occasion, the flexible board 6 extending from the imaging board 41 towards the control board 51 is made to turn to the side of the board receiver 300c.

At step S34, the control board 51 is attached to the first spacer divided body 310. Specifically, the lug part 51a of the control board 51 is made to engage with the board rotation regulating part 310d and the control board 51 is inserted to the board engagement part 310c from the lateral direction.

At step S35, the second spacer divided body 320 is attached to the first spacer divided body 310. Specifically, the second spacer divided body 320 is made to engage with the first spacer divided body 310 in a manner of lodging the imaging board 41 and the control board 51 from the lateral direction. Then, the boss 310e of the first spacer divided body 310 and the fitting hole 320f of the second spacer divided body 320 are fitted and the boss 320e of the second spacer divided body 320 and the fitting hole 310f of the first spacer divided body 310 are fitted. It is preferable that the lens frame engagement parts 310a and 320a and the spacer fitting part 205g are fixed by an interference fit for settling a position in the axial direction of the lens frame 205 with respect to the spacer 300.

At step S36, the illumination board installation part 205i of the lens frame 205 is inserted to the opening 31a of the illumination board 31 and temporarily fixed. Here, the illumination board 31 connected to the flexible board 6 extending from the imaging board 41 is fixed in position with respect to the spacer 300 due to the attachment of the dome part 10. Therefore, the position of the illumination board 31 is not completely settled because of the restoring force of the flexible board 6 at this stage. In this embodiment, a length of the flexible board 6 between the imaging board 41 and the illumination board 31, a diameter of the illumination board installation part 205i, and a diameter of the opening 31a are coordinated appropriately to prevent the illumination board 31 from coming off from the illumination board installation part 205i. Specifically, the flexible board 6 between the imaging board 41 and the illumination board 31 is adjusted not to be too long, so that the restoring force of the flexible board 6 is controlled to work obliquely upward with respect to the illumination board 31. Thus, it is possible to prevent the illumination board 31 from coming off from the illumination board installation part 205i since the inner circumference of the opening 31a gets stuck with the illumination board installation part 205i.

Next, the battery unit 4 will be explained in detail.

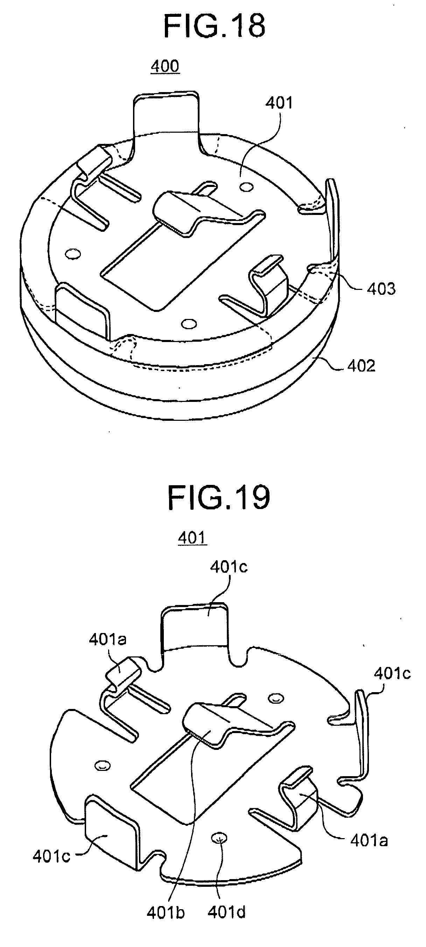

FIG. 18 is a perspective view of an external appearance of the first battery lot 400. The first battery lot 400 is provided with a battery 402 having a disk-like shape (button-like shape), a positive electrode contact point member 401 that enables obtaining conduction by being in contact with a positive electrode surface of the battery 402, and a fastening member 403 that unites the battery 402 and the positive electrode contact point member 401.

FIG. 19 is a perspective view of a structure of the positive electrode contact point member 401.

The positive electrode contact point member 401 is a metal member which is, for example, about as thick as 0.1 mm and has spring characteristics. At two locations facing in the outer circumferential part of the positive electrode contact point member 401, spacer engagement parts 401a used in retaining the spacer 500 are provided in a manner of facing with each other.

In the vicinity of a center part on a bottom surface of the positive electrode contact point member 401, a board contact point part 401b that depresses the wireless board 61 and enables an electrical connection between the battery 402 and the wireless board 61 is provided. The board contact point part 401b is a tongue-like piece which protrudes and extends to the side of the wireless board 61 when assembled to the casing, and has spring characteristics of depressing the wireless board 61 when assembled.

In the outer circumferential part of the positive electrode contact point member 401, three L-shaped bent parts 401c are provided in a manner of rising up at 90 degrees on the basis of the bottom surface of the positive electrode contact point member 401. One of the L-shaped bent parts 401c is provided at a location facing the board contract point part 401b. Besides, the other two of the L-shaped bent parts 401c are provided at plane-asymmetrical locations across the board contact point part 401b.

At a plurality of locations (three locations in FIG. 19) on the bottom surface of the positive electrode contact point member 401, convex parts 401d protruding toward a contact surface with the battery 402 are formed. These protruding parts 401d allow securing conduction between the positive electrode contact point member 401 and the battery 402.

The positive electrode contact point member 401 is formed by a press molding process using a metal thin plate having spring characteristics such as a stainless steel (SUS304CSP, for example) and a phosphor bronze (C5210P, for example), and then manufactured via a base process using Ni-P plating approximately as thick as 3 µm and a surface-finishing process using Au-Co plating approximately as thick as 0.5 µm, for example.

The fastening member 403 is formed by a heat-shrinkable tube of polyethylene terephthalate, polyvinyl chloride, and the like which shrink by heating. Specifically, it is preferable to use a material which shrinks by a short time heating at as high as 80 degrees C to suppress a thermal influence on the battery 402. Besides, it is preferable to use a material which is as thick as 0.05 to 0.1 mm after the shrinkage to suppress an increase in diameter of the first battery lot 400.

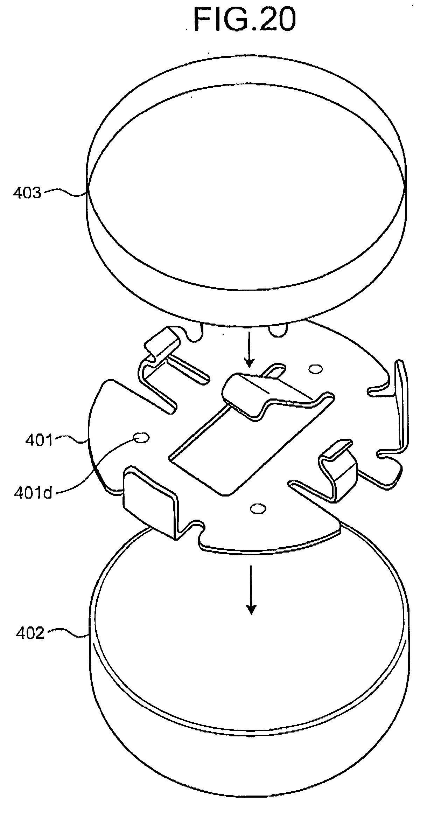

Next, a method for manufacturing the first battery lot 400 will be explained. First, as shown in FIG. 20, the positive electrode contact point member 401 is arranged at the positive electrode surface side of the battery 402 so that the convex part 401d protrudes to the side of the battery 402. Then, the fastening member 403 is put in a manner of covering the peripheral part of the positive electrode contact point member 401 and a part of the side surface of the battery 402. On this occasion, a cutout and the like may be provided in the fastening member 403 in accordance with the shape of the positive electrode contact point member 401. Besides, it is preferable to arrange the fastening member 403 so that the fastening member 403 continues from the peripheral part of the positive electrode contact point member 401 to the side surface of the battery 402 at least at three positions. By heating and causing the fastening member 403 to shrink in this state, the battery 402 and the positive electrode contact point member 401 are united. Thus, the first battery lot 400 shown in FIG. 18 is completed.

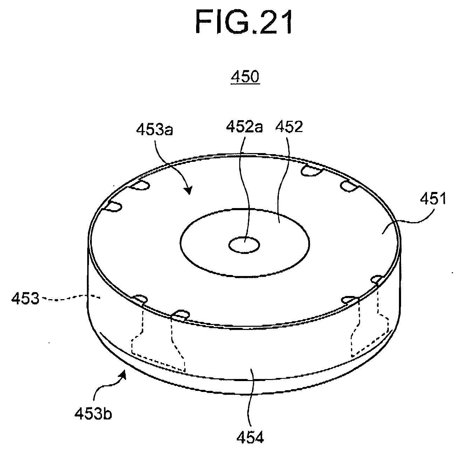

FIG. 21 is a perspective view of an external appearance of the second battery lot 450. The second battery lot 450 is provided with the battery 453 having a disk-like shape (button-like shape), an insulation sheet 451 that prevents a short circuit in the battery 402 of the first battery lot 400 in the assembling, a contact point plate 452 that provides a contact point between the battery 453 and the first battery lot 400, and a fastening member 454 that unites the battery 453, the insulation sheet 451, and the contact point plate 452.

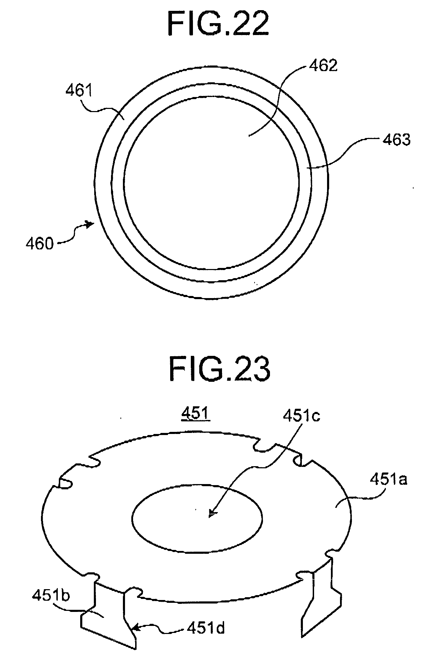

Here, as shown in FIG. 22, a negative electrode surface side of the battery 460 having a disk-like shape generally has a structure in which a battery positive electrode can 461 extending from the positive electrode surface at the other side and a negative electrode 462 provided inside are separated by a separator 463. Therefore, in the case where the negative electrode side of the first battery lot 400 (negative electrode surface of the battery 402) and the positive electrode side of the second battery lot 450 (positive electrode surface of the battery 453) are made to face in the arrangement as shown in FIG. 1, there is a possibility of causing a short circuit in response to a conduction between the battery positive electrode can and the negative electrode of the battery 402 via the positive electrode surface of the battery 453 when the facing surfaces are left exposed. In this embodiment, an occurrence of the short circuit is prevented by arranging the insulation sheet 451 at the positive electrode surface side of the battery 453.

FIG. 23 is a perspective view of the insulation sheet 451. The insulation sheet 451 is provided with a circular part 451a having an outer diameter equivalent to the outer diameter of the battery 453 and a predetermined number of (four, for example) extension parts 451b extending from the circular part 451a to its periphery side. Nearly at the center part of the insulation sheet 451, an opening part 451c that allows a convex part 452a of the contact point plate 452 to be exposed is formed and the insulation sheet 451 covers the positive electrode surface of the battery 453 in an area other than this opening part 451c.

The extension parts 451b are provided at predetermined intervals (90 degrees, for example) in the periphery of the circular part 451a. These extension parts 451b are sandwiched between the side surface of the battery 453 and the fastening member 454 and retain the contact point plate 452 in a manner of enclosing it with respect to the battery 453.

Edge part of each extension part 451b has a spatula shape in which a distal edge becomes broadened. By this configuration, an area of a part to be sandwiched between the side surface of the battery 453 and the fastening member 454 becomes large and the insulation sheet 451 is therefore retained stably. It is preferable to provide a slope part 451d inclined at about 45 degrees in a part of the spatula shape. Thus, it is possible to arrange the extension parts 451b in a manner of being along the side surface of the battery 453 without causing a fold and the like in the extension parts 451b in covering the battery 453 with the fastening member 454.

The insulation sheet 451 is manufactured by performing a press punch-out process on, for example, a polyimide film (Kapton® film, manufactured by DU PONT-TORAY CO., LTD., for example).

Nearly at the center part of the contact point plate 452, the convex part 452a is formed. The contact point plate 452 obtains a conduction by being in contact with the negative electrode surface of the battery 402 of the first battery lot 400 in the convex part 452a and also a conduction by being in contact with the positive electrode surface of the battery 453 on a surface at the opposite side of the surface where the convex part 452a is formed. A height of the convex part 452a is determined so that a summit becomes higher than the thickness of the insulation sheet 451. Besides, it is preferable that a diameter of the convex part 452a is made as small as possible in a standing part from the contact point plate 452 to enhance a rigidity of the convex part 452a.

The contact point plate 452 is formed by a press molding process using a phosphor bronze (C5191) which is easily made thin, for example, and further manufactured via a base process using Ni-P plating approximately as thick as 0.5 µm and a surface-finishing process using Au-Co plating approximately as thick as 0.5 µm, for example.

The fastening member 454 is formed by a heat-shrinkable tube of polyethylene terephthalate, polyvinyl chloride, and the like similarly to the fastening member 403. It is preferable that the fastening member 454 is arranged in a manner of being slightly lower than an upper surface of the insulation sheet 451 at the side of the positive electrode surface 453a to prevent an edge part of the fastening member 454 from extending to the positive electrode surface 453a in the state after being shrunk. Thus, it becomes unnecessary to provide a back clearance for the fastening member 454 between the battery 453 and the battery 402 after the assembling and it is possible to promote a space saving. It is also preferable that the fastening member 454 is looped around in a manner of at least covering a part of the battery positive electrode can extending at the side of the negative electrode surface 453b. Thus, it is possible to prevent an occurrence of a short circuit caused by a contact of other metal parts with the side of the negative electrode surface 453b in the assembling and the like.

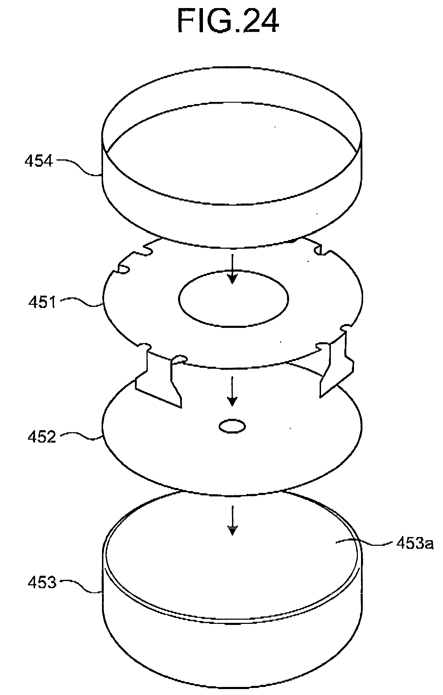

Next, a method for manufacturing the second battery lot 450 will be explained. First, the contact point plate 452 and the insulation sheet 451 are arranged sequentially at the side of the positive electrode surface 453a of the battery 453 as shown in FIG. 24. Then, the fastening member 454 is put in a manner of covering the side surface of the battery 453. On this occasion, it is preferable to put the fastening member 454 in a manner of pushing the extension parts 451b of the insulation sheet 451 against the side surface of the battery 453. Thus, it becomes unnecessary to prepare a bending habit and the like in the insulation sheet 451 in advance since the slope part 451d provided in the extension part 451b is bent into a shape along the inner wall by being in contact with the inner wall of the fastening member 454.

By heating and causing the fastening member 454 to shrink in this state, the battery 453, the contact point plate 452, and the insulation sheet 451 are united. Thus, the second battery lot 450 shown in FIG. 21 is completed.

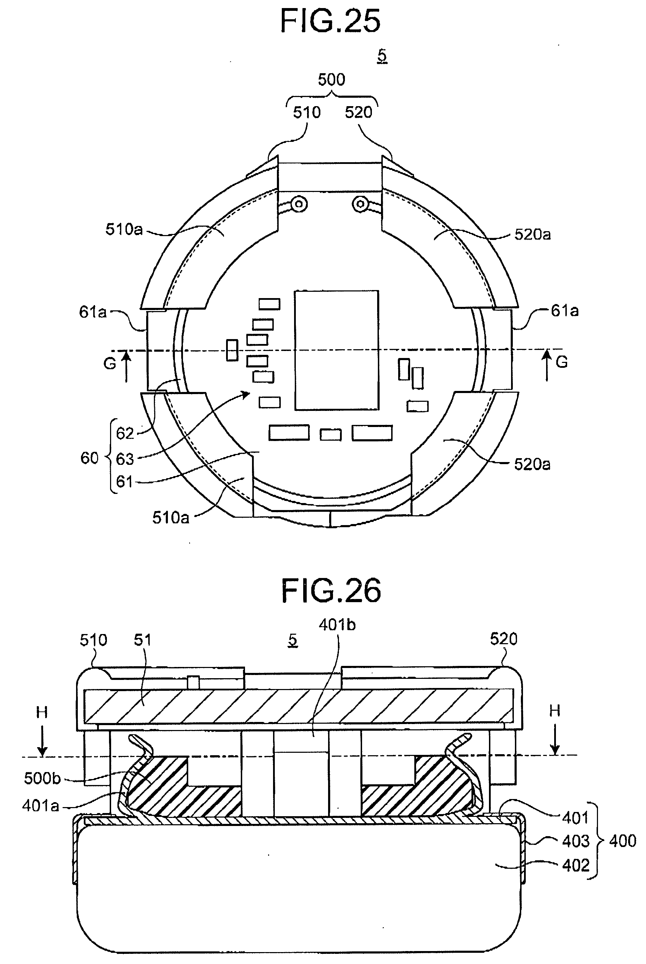

Next, the second block part 5 will be explained in detail. FIG. 25 is a top view of an external appearance of the second block part 5 seen from a side of a surface on which the transmission antenna 62 of the wireless communication unit 60 is formed. Besides, FIG. 26 is a cross sectional view along the line G-G in FIG. 25. In FIG. 26, the first battery lot 400 attached to the second block part 5 is shown together.

As shown in FIG. 25, the spacer 500 has a cylindrical contour as a whole and is constituted by a pair of divided bodies (a first spacer divided body 510 and a second spacer divided body 520) which are separated by a plane passing through a central axis of the cylindrical shape. The first spacer divided body 510 and the second spacer divided body 520 retain, by lodging therebetween, the wireless communication unit 60.

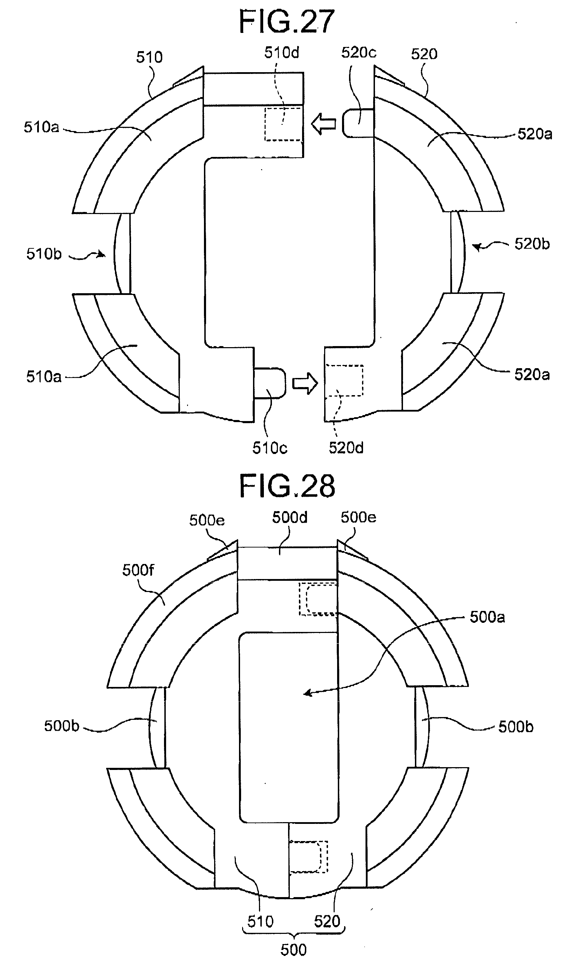

FIG. 27 shows a state in which the first spacer divided body 510 and the second spacer divided body 520 are separated. In the first spacer divided body 510, a wireless board engagement part 510a that supports the wireless board 61 from the lateral direction is formed at two locations on the arc. A cross section of the wireless board engagement part 510a has an orthogonally inclined U-shape for lodging the wireless board 61. Between these wireless board engagement parts 510a, a wireless board rotation regulating part 510b that regulates a positional displacement in the rotating direction of the wireless board 61 in engagement with the lug part 61a of the wireless board 61 is provided.

Besides, the first spacer divided body 510 is provided with a boss 510c and a fitting hole 510d as a fitting part for being united with the second spacer divided body 520.

In the second spacer divided body 520, a wireless board engagement part 520a that supports the wireless board 61 from the lateral direction is formed at two locations on the arc. A cross section of the wireless board engagement part 520a has an orthogonally inclined U-shape for lodging the wireless board 61. Between these wireless board engagement parts 520a, a wireless board rotation regulating part 520b that regulates a positional displacement in the rotating direction of the wireless board 61 in engagement with the lug part 61a of the wireless board 61 is provided.

Besides, the second spacer divided body 520 is provided with a fitting hole 520d formed at a position facing the boss 510c of the first spacer divided body 510 and a boss 520c formed at a position facing the fitting hole 510d.

As shown in FIG. 28, a board contact point opening 500a is provided in the vicinity of a center part of the spacer 500. The board contact point opening 500a is a space in which the board contact point part 401b of the positive electrode contact point member 401 that electrically connects the first battery lot 400 and the wireless board 61 is arranged as shown in FIG. 26. In the board contact point opening 500a, the board contact point part 401b standing from the positive electrode contact point member 401 has a contact with the pad 64 formed in the flexible board 6 at the rear side of the wireless board 61. Thus, it is possible to arrange the wireless board 61 and the battery unit 4 with a certain distance away from each other without increasing the number of folds of the flexible board 6.

Here, it is preferable that a length in the axial direction of the board contact point opening 500a (i.e., a length from the rear surface of the wireless board 61 to the edge face of the spacer 500 at the side of the battery unit 4) is designed based on an intensity of a wireless signal transmitted from the wireless communication unit 60, a size of the battery unit 4, and the like. The reason is that it becomes possible to electrically connect the wireless board 61 and the battery unit 4 with a distance necessary for obtaining predetermined wireless characteristics away from each other by designing the length of the board contact point opening 500a appropriately in such a manner as the embodiment though emission characteristics of a wireless signal normally deteriorate when a metal member is arranged in the vicinity of a source of emission of the wireless signal.

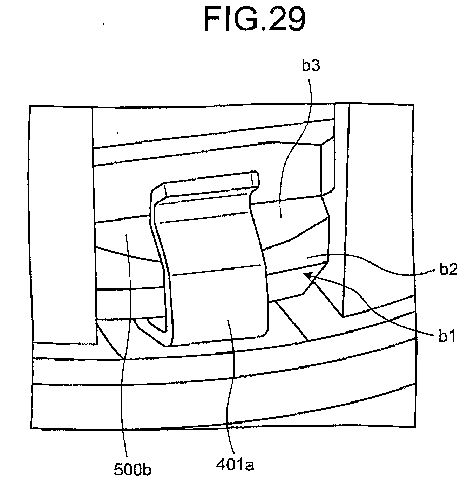

Besides, two electrode contact point member engagement parts 500b to which the two respective spacer engagement parts 401a formed on the positive electrode contact point member 401 are fixed are formed at positions facing with each other in the circumferential part of the spacer 500. FIG. 29 is an enlarged perspective view of the positive electrode contact point member engagement part 500b. As shown in FIG. 29, for a shape of the positive electrode contact point member engagement part 500b, it is preferable that a surface b1 is designed to be a slope surface forming a part of a conical surface, a surface b2 is designed to be a curved surface forming a part of a side surface of a cylinder, and a surface b3 is designed to be an inclined plane to make it easy to fit the spacer engagement part 401a with the positive electrode contact point member engagement part 500b.

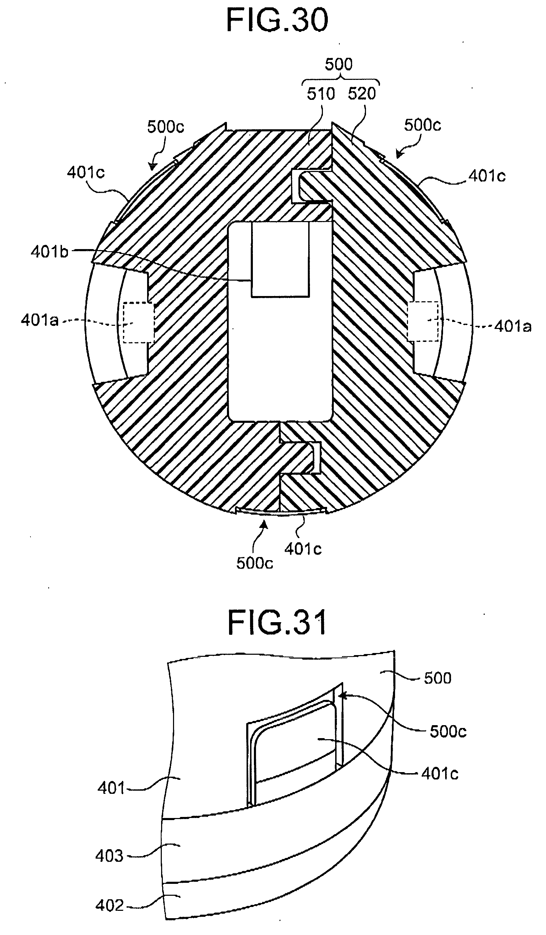

FIG. 30 is a cross sectional view of a case in which the spacer 500 is cut, as a cross sectional surface, along the line H-H in FIG. 26. As shown in FIG. 30, a positive electrode contact point member position regulating part 500c that is formed by cutting off a part of the circumference is provided at three locations.

FIG. 31 is an enlarged perspective view of the vicinity of the positive electrode contact point member position regulating part 500c and shows a state before the positive electrode contact point member 401 is fitted. As shown in FIG. 31, the positive electrode contact point member position regulating part 500c is a part that engages with the L-shaped bent part 401c formed in the positive electrode contact point member 401. By this configuration, the axial rotation of the positive electrode contact point member 401 with respect to the spacer 500 is regulated when fitted with the spacer 500.

The board receiver 500d is formed in the circumferential part of the spacer 500. The board receiver 500d is a part which allows arranging the flexible board 6 extending from the wireless board 61 retained by the spacer 500 by letting it get out of the spacer 500 once without lodging it between the first spacer divided body 510 and the second spacer divided body 520. By letting the flexible board 6 get out of the spacer 500 in this manner, it is possible to suppress an excessive fold of the flexible board 6 and thereby to reduce damage. A round chamfering is made along an edge line, where the flexible board 6 contacts, of the flexible board receiver 500d.

At both sides of the flexible board receiver 500d, a rotation regulating part 500e that protrudes outward from the circumference is formed. The rotation regulating part 500e is a part which engages with the case groove part 20d when the second block part 5 is housed in the case part 20, and has nearly triangular shape in accordance with the case groove part 20d. Thus, the rotation of the second block part 5 in inserting the second block part 5 into the case part 20 is prevented.

In the outer circumference at a distal end side (a side to be arranged at the hemisphere side of the case part 20) of the spacer 500, a large round chamfering part 500f is provided. Thus, the round chamfering part 500f is in contact with a round part of the inner wall of the case hemispherical part 20a and the spacer 500 is automatically centered with respect to the case part 20 when the second block part 5 is inserted into the case part 20.

The spacer 500 (the first spacer divided body 510 and the second spacer divided body 520) is formed via an injection molding using resin materials such as polycarbonate, acrylonitrile-butadiene-styrene, polyoxymethylene (POM), and modified polyphenylene ether (PPE; modified PPO), for example. Especially, since being light in weight and of an adequate mechanical strength compared to other resins, the modified PPO is advantageous in that a crack hardly occurs in the assembled state and the like.

The second block part 5 is assembled in such a manner as explained below. First, the wireless board 61 on which the electronic part 63 is mounted is attached to the first spacer divided body 510. On this occasion, the lug part 61a of the wireless board 61 is made to engage with the wireless board rotation regulating part 510b and the wireless board 61 is inserted to the wireless board engagement part 510a from the lateral direction. Besides, the flexible board 6 extending from the wireless board 61 is arranged to locate at the side of the board receiver 500d.

The second spacer divided body 520 is next made to engage from the lateral direction in a manner of lodging the wireless board 61 with the first spacer divided body 510. Then, the boss 510c of the first spacer divided body 510 and the fitting hole 520d of the second spacer divided body 520 are fitted and the boss 520c of the second spacer divided body 520 and the fitting hole 510d of the first spacer divided body 510 are fitted.

Next, a method for assembling the first battery lot 400 to the second block part 5 will be explained.

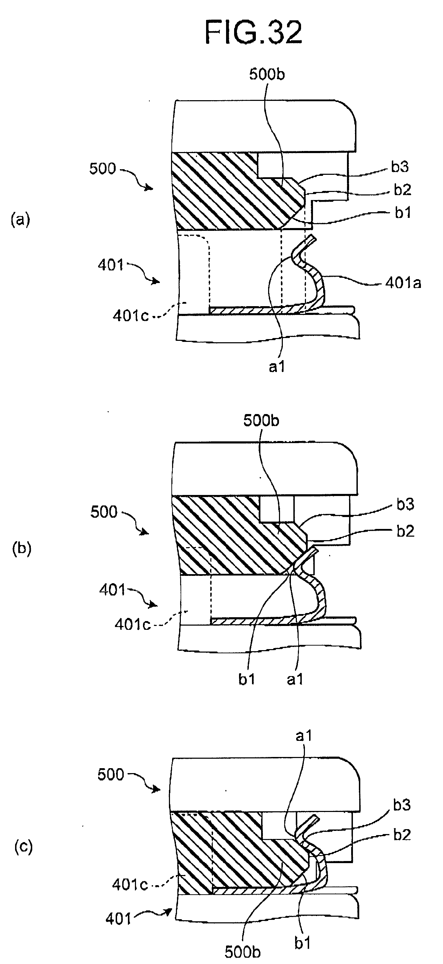

First, a position in the rotational direction is adjusted by using, as a land mark, the three L-shaped bent parts 401c provided in the positive electrode contact point member 401 of the first battery lot 400 and the three positive electrode contact point member position regulating parts 500c provided in the spacer 500, as shown in FIG. 32(a).

Next, the positive electrode contact point member engagement part 500b is made close to the spacer engagement part 401a of the positive electrode contact point member 401 as shown in FIG. 32(b). As shown in FIG. 32(b), the spacer engagement part 401a becomes stretched out along the surface b1 when the positive electrode contact point member engagement part 500b becomes in contact with the spacer engagement part 401a. When the positive electrode contact point member engagement part 500b is further depressed to the spacer engagement part 401a, the spacer engagement part 401a returns, after passing through the surface b2, to the original shape along the surface b3 on its own elastic force (see FIG. 32(c)). Thus, the spacer 500 and the first battery lot 400 are retained by the positive electrode contact point member 401.

It is preferable to depress the spacer 500 while keeping the spacer 500 parallel to the positive electrode contact point member 401. This is because the L-shaped bent part 401c can be prevented from being deformed due to a movement or an inclination of the spacer 500 beyond a certain limit since the periphery of the three positive electrode contact point member position regulating parts 500c comes to be in contact with the inner circumference of the three L-shaped bent parts 401c almost at the same time.

Here, a relation in shape between the positive electrode contact point member engagement part 500b and the positive electrode contact point member 401 and the spring characteristics will be explained. An inflection part summit a1 is arranged within a range between both ends of the surface b1 in the radial direction so that the spacer engagement part 401a is stretched out naturally when the positive electrode contact point member engagement part 500b comes to be in contact and its restoring force arises on the surface b3 when the positive electrode contact point member engagement part 500b is further depressed.

While the two spacer engagement parts 401a clamp the spacer 500 to the direction of depressing the bottom surface of the positive electrode contact point member 401, the board contact point part 401b generates a force depressing the wireless board 61 to the side of the spacer 500 against the clamping force. Therefore, it is preferable to adjust spring characteristics in the positive electrode contact point member 401 so that a resultant force of the clamping force of the two spacer engagement parts 401a becomes larger than the reaction force by the board contact point part 401b. Thus, it is possible to retain the spacer 500 with respect to the positive electrode contact point member 401 while keeping the state of depressing the board contact point part 401b to the wireless board 61.

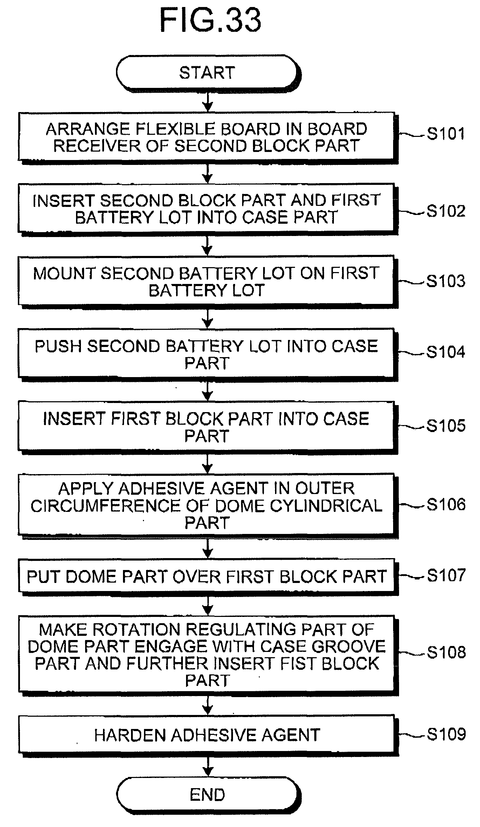

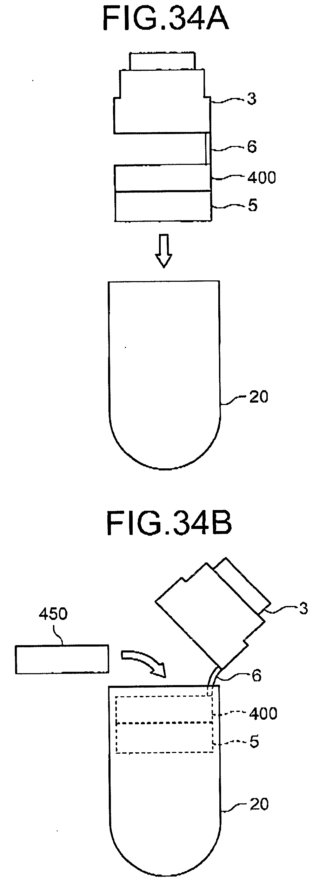

A method for manufacturing the capsule medical apparatus 1 will be explained next with reference to FIGS. 33 and 34A to 34E.

First, the flexible board 6 is made to extend from an area between the two rotation regulating parts 500e of the spacer 500 and arranged so that a fold part of the flexible board 6 is put along the round chamfering part of the board receiver 500d (step S101).

The second block part 5 to which the first battery lot 400 is attached is then inserted into the case part 20 (step S102) as shown in FIG. 34A. On this occasion, a state in which the rotation regulating parts 500e of the spacer 500 is made to engage with the case groove part 20d on the inner wall of the case part 20 is maintained.

Then, the second battery lot 450 is mounted, in a manner of making its positive electrode surface in direct contact, on the negative electrode surface of the first battery lot 400 when the negative electrode surface of the first battery lot 400 is inserted to the inner side of the case edge part 20g as shown in FIG. 34B (step S103).

By pushing the second battery lot 450 to the inside of the case part 20, the second battery lot 450, the first battery lot 400, and the second block part 5 are further inserted into the case part 20 (step S104).

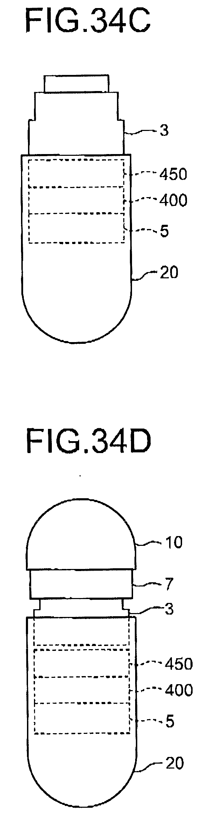

As shown in FIG. 34C, the center of the first block part 3 and the center of the case part 20 are set and the first block part 5 is inserted into the case part 20 when the negative electrode surface of the second battery lot 450 is inserted to the inner side of the case edge part 20g (step S105).

The inserting operation is once stopped when the end part of the first block part 3 reaches the case edge part 20g, the dome grasping part 10b of the dome part 10 is grasped by a pair of tweezers and the like, and the adhesive agent 7 of the thermoset type or the UV cure type is applied on the outer circumferential surface of the dome cylindrical part 10c (step S106).

After fitting the position of the rotation regulating part 10e and the position of the case groove part 20d, the dome part 10 is then put over the first block part 3 protruding from the case edge part 20g as shown in FIG. 34D to make the dome cylindrical part 10c engage with the dome fitting part 300a (step S107). Then, the first block part 3 is biased and inserted until the case edge part 20g comes to be in abutting contact with the edge face 10d of the dome part 10 (step S108).

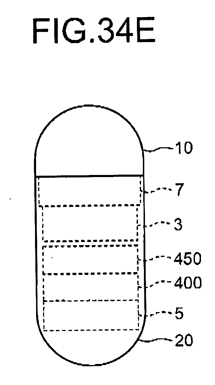

The adhesive agent 7 is hardened in this state while applying a load in the longitudinal direction of the casing 2 so that the dome part 10 does not move up from the case part 20 (step S109). Thus, the capsule medical apparatus 1 shown in FIG. 34E is completed.

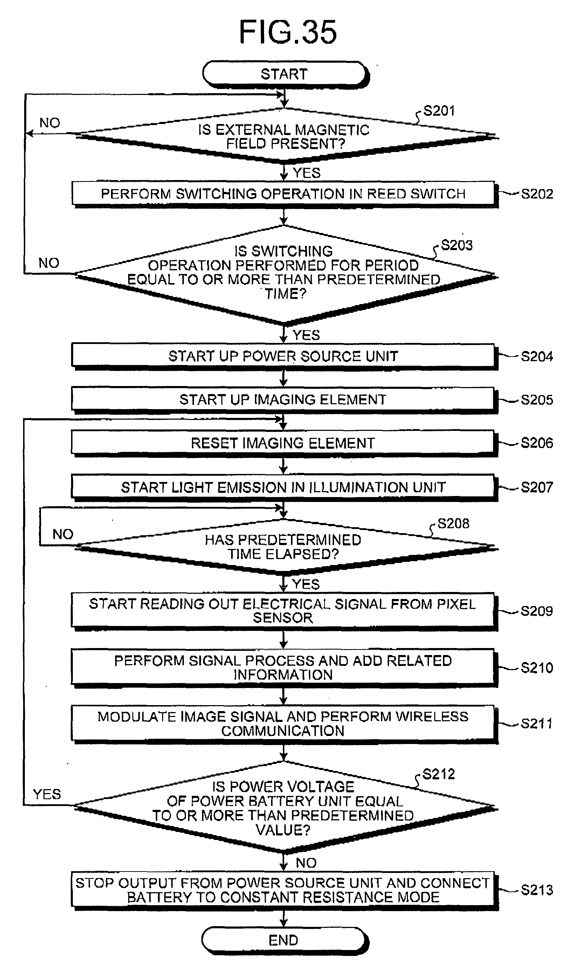

Next, an operation of the capsule medical apparatus 1 will be explained with reference to FIGS. 2 and 35. FIG. 35 is a flowchart of an operation of the capsule medical apparatus 1.

When a magnetic field is externally applied on the capsule medical apparatus 1 by using a magnet and the like ("Yes" at step S201), the reed switch 52 responds to the external magnetic field to perform a switching operation (step S202). When the switching operation is performed for a period equal to or more than a predetermined time ("Yes" at step S203), the power source controller 53a starts up the power source unit 53b (step S204). On the other hand, when the external magnetic field is not applied ("No" at step S201), or when the period for which the external magnetic field is applied is shorter than the predetermined time ("No" at step S203), the power source controller 53a waits for the application of the external magnetic field again on the capsule medical apparatus 1.

The imaging controller 43a then sets the internal register 43c based on operation setting information stored in the memory 54 and starts up the imaging element 42 (step S205). Besides, the imaging controller 43a discharges and resets an electric charge stored in each pixel sensor of the imaging element 42 before making the illumination unit 30 emit a light (step S206).

The illumination driver 53c then supplies a power to the illumination unit 30 to start the emission of light (step S207). On this occasion, it is necessary to start up the emission of light of the illumination unit 30 precipitously for performing a light modulating control by making a light amount and a light emission time of the illumination unit 30 have a proportional relation. To this end, it is preferable to make a voltage high in advance before the emission of light and to start applying an electric current at the moment of the start of the emission of light.

After the start of the emission of light by the illumination unit 30, the imaging controller 43a waits for an elapse of a predetermined time ("No" at step S208). When the predetermined time elapses ("Yes" at step 208), the imaging controller 43a then starts reading out an electrical signal from a pixel sensor (step S209). Here, the reason of the wait for the elapse of the predetermined time after the start of the emission of light by the illumination unit 30 is that a signal is read out after power voltage returns and becomes stable since the power voltage temporarily drops due to the light emission by the illumination unit.

The signal processor 43b performs a signal process on the electrical signal read out from the pixel sensor to generate an image signal and also adds related information to the image signal (step S210). Specific signal processes includes a non-linearization process that reduces the bit count of the electrical signal. Besides, the related information includes information of the light emission time of the illumination unit 30, ID information, stored in the internal register 43c, of the capsule medical apparatus 1, and the like. The related information is added repetitively three times, for example, to improve a tolerance for noise.

The modulator 63a modulates the image signal output from the signal processor 43b and has the image signal transmitted wirelessly via the transmission antenna 62 (step S211).

The power source controller 53a checks whether or not the power voltage of the battery unit 4 is not less than a predetermined value (step S212). When the power voltage is not less than the predetermined value ("Yes" at step S212), the operation returns to step S206. On the other hand, when the power voltage is less than the predetermined value ("No" at step S212), the power source controller 53a stops an output from the power source unit 53b and connects the battery to a constant resistance mode (step S213).

As explained so far, since the assembling is performed by inserting a blocked group of parts constituting the capsule medical apparatus into the casing 2 of the capsule, it is possible to reduce the number of parts dealt with in the assembling and perform the assembling operation easily and in a short time.

Besides, since respective surfaces which come to be in abutting contact with each other are provided in the first to the third lenses 201 to 203 and surfaces to be fitted with the lens frame 205 are provided in the objective lens unit 200, it becomes possible according to the embodiment to determine the position of parts both in the axial direction and in the radial direction only by putting parts down in series to the lens frame 205. Hence, it becomes unnecessary to perform a focus adjustment and an axial adjustment of the objective lens unit 200. It also becomes unnecessary to have a member that defines a distance and the like in lens surfaces like the conventional techniques, resulting in a reduction of the number of parts. Besides, since the assembling operation becomes simple and easy, it becomes possible to reduce factors, such as a lens damage and a dust adherence, that degrade the optical performance during the operation.

Moreover, it is possible according to the embodiment to uniquely determine the distance between the third lens 203 and the imaging element 42 since the imaging element abutting part 203f extending from the third lens 203 is made to abut directly on the imaging element 42. Hence, it becomes unnecessary to perform an operation for a focus adjustment and an axis adjustment with respect to the imaging element 42. Besides, since the outer side of the imaging element abutting part 203f is sealed by an adhesive agent, it becomes unnecessary to arrange a glass plate and the like that protect the light-receiving surface of the imaging element 42 and it becomes possible to reduce the number of parts and to make the assembling operation simple and easy.

Besides, it becomes possible according to the embodiment to enhance durability by reducing the number of fold parts of the flexible board 6.

The functional units embedded in the capsule medical apparatus are not limited to those explained in the embodiment above. For example, so-called binocular capsule medical apparatus in which two imaging units are arranged at both sides of the capsule shaped casing may be adopted. It is possible in this case, too to perform the assembling operation easily by arranging the two imaging units as respective blocks.

Moreover, the number of batteries to be embedded in the capsule medical apparatus is not limited to two as explained in the above embodiment. For example, in a case of providing one battery, it is only necessary to omit the second battery lot 450 and to use only the first battery lot 400. In this case, the first battery lot 400 is directly connected to the negative electrode contact point spring 480. Alternatively, it is also possible to provide three or more batteries by increasing the number of the second battery lot 450. In this case, it is only necessary to insert desired number of the second battery lots 450 between the first battery lot 400 and the first block part 3.

### First modification

Next, a modification example of the shape of the illumination board 31 which is flexible and integrally formed with the flexible board 6 will be explained.

The illumination board 31 shown in FIG. 2 has a shape obtained by cutting two parts of a periphery of a circular shape by D-shape. The D-shape cutting allows the flexible board 6 extending from the illumination board 31 to be easily folded from a root part of the flexible board 6 (from a connection part with the illumination board 31). However, due to this shape, there is a case where the illumination board 31 cannot be held by the edge face 10d sufficiently since an area of the illumination board 31 to be in contact with the edge face 10d of the dome cylindrical part 10c becomes small when the illumination unit 30 and the like are housed in the case part 20 and covered by the dome part 10. As a result of this, there is a possibility that the illumination board 31 moves up from a home position and a positional regulation of each illumination element 32 becomes unstable.

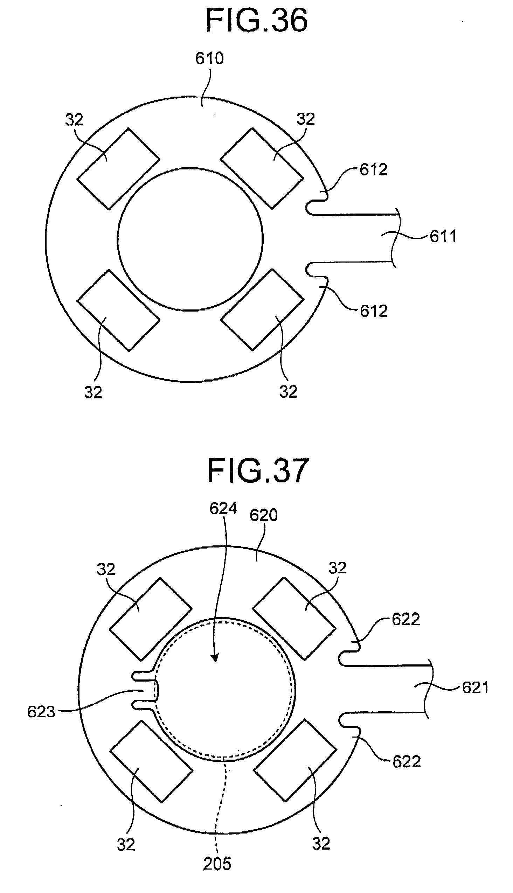

In response to this case, an entirety of an illumination board 610 is formed in a circular shape and a part at both sides of the flexible board part (extension part 611) extending from the illumination board 610 is cut in to form a lug part 612 as shown in FIG. 36, for example in the first modification. This allows the extension part 611 to be easily folded from the root part and a contact area of the illumination board 610 with the edge face 10d of the dome cylindrical part 10c to increase. As a result of this, it becomes possible to hold the illumination board 610 sufficiently by the edge face 10d and keep the position of each illumination element 32 stably.

### Second modification

Next, another modification example of the shape of the illumination board 31 will be explained.

While the illumination board 31 is retained in the state of making the objective lens unit 200 (lens frame 205) inserted into the opening 33 in assembling the illumination unit 30 to the first block part 3, the position of the illumination board 31 itself is not fixed. Therefore, in inserting the first block part 3 to the inside of the case part 20 and putting the dome part 10 over it, there arises a necessity that an operator should work while holding the illumination board 31 and there is a possibility of causing a degradation in workability and an increase in the operation time.

In response to this, an entirety of an illumination board 620 is formed in a circular shape, a lug part 622 is provided at both sides of an extension part 621, and a tongue part 623 whose tip end protrudes from an inner circumference to an inner circle side is also provided at the inner circumference side of the circular shape as shown in FIG 37, for example in the second modification. A cut is provided at both sides of the tongue part 623, which enables the tongue part 623 to deform in a manner of flipping upward in a direction perpendicular to the surface of the illumination board 620. It is only necessary that the tongue part 623 protrudes to the side of the inner circle to such an extent that the tongue part 623 adequately contacts the outer circumference of the lens frame 205 to be inserted to an opening 624.

When the illumination board 620 is put over the lens frame 205, the tongue part 623 having deformed along the lens frame 205 tends to return to the original planar shape and a force works in a direction of bearing the lens frame 205 down. A frictional resistance generated due to the force between the tongue part 623 and the lens frame 205 allows suppressing coming-off of the illumination board 620 from the lens frame 205. As a result of this, it becomes possible to enhance workability in the assembling operation of the capsule medical apparatus 1 and shorten the operation time.

Here, it is possible to adjust a force (i.e., strength of the frictional resistance) of suppressing the coming-off of the illumination board 620 by the tongue part 623 by a protrusion amount by which the tongue part 623 protrudes to the inner circle side from the circumference of the opening 624 and a distance from a tip end of the tongue part 623 to the root part (end part of the cut part). Besides, while the position where the tongue part 623 is provided is not limited, it is preferable to provide it at a side opposite to the extension part 621 as shown in FIG. 37 since the position of the illumination board 620 can be regulated efficiently. Furthermore, the number of the tongue part 623 is not limited and two or more tongue parts may be provided.

### Third modification

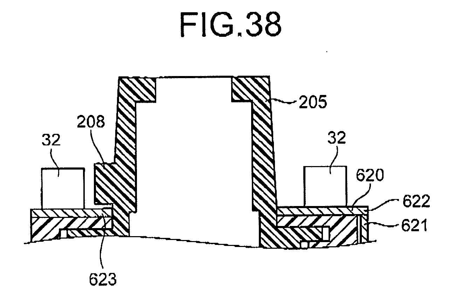

In the case of using the illumination board 620 provided with the tongue part 623 explained in the second modification, a protruding part 208 may be provided at a position, interfering with the tongue part 623, in the outer circumference of the lens frame 205 (i.e., the outer circumference at the side opposite to the extension part 621) as shown in FIG. 38. In this case, it becomes possible to surely prevent the coming-off of the illumination board 620 from the lens frame 205.

### Fourth modification

Next, another modification example of the shape of the illumination board 31 will be explained.

There is a case where the illumination board 31 is temporarily fixed for positioning by using a double-sided adhesive tape, an adhesive agent, and the like with respect to the lens frame 205 in assembling the illumination unit 30 to the first block part 3. However, an additional man-hour for using the adhesive agent and the like is required in the case of performing the temporal fixation. Besides, since the adhesive agent and the like are required to be removed when a mistake is made in the positioning, there is a difficulty in making another try and there is also a possibility of causing a trouble and degrading a yield ratio.

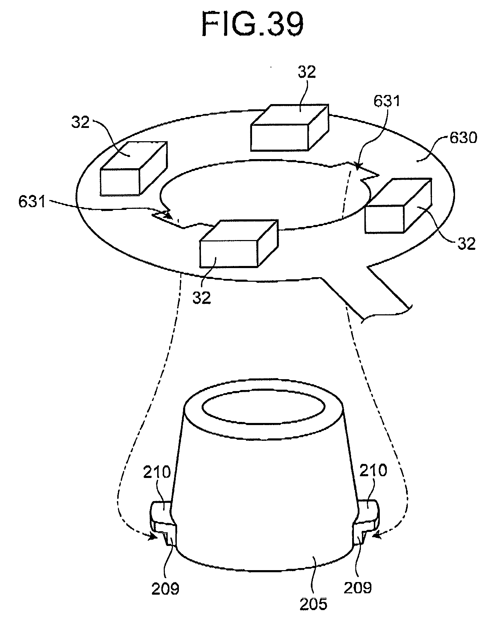

In response to this, at least one (two in FIG. 39) guiding part 209 protruding outward is provided in the outer circumference of the lens frame 205 and a nail part 210 further protruding from the guiding part 209 is provided at the upper part of the guiding part 209 as shown in FIG. 39, for example in the fourth embodiment. In an illumination board 630, an entirety is formed in a circular shape and at least one (two in FIG. 39) concave part 631 is provided by cutting partially a position, corresponding to the guiding part 209, in the inner circumference. Then, the illumination board 630 is put over the lens frame 205 in a manner of fitting the concave part 631 with the guiding part 209 and the concave part 631, after passing through the nail part 210, is fitted with the guiding part 209. Thus, a movement in the circumferential direction of the illumination board 630 is regulated and the coming-off of the illumination board 630 from the lens frame 205 is suppressed by the nail part 210.

According to the fourth modification, it is possible to fix the illumination board 630 to the lens frame 205 easily and temporarily without using the adhesive agent and the like. Hence, it become possible to reduce the man-hour for the temporal fixation and to make another try for the positioning easily. As a result of this, it becomes possible to suppress an occurrence of a trouble due to a retry for the positioning.

### Fifth modification

Next, a modification example of the spacer 300 will be explained.

There is a case where a board part 640 is temporarily fixed to the spacer 300 by using a double-sided adhesive tape, an adhesive agent, and the like in positioning the board part 640 which is flexible and on which the negative electrode contact point spring 480 shown in FIG. 2 is mounted with respect to the spacer 300. However, there are problems that an additional man-hour is required in this case, too and that it is difficult to make another try when a mistake is made in the positioning.

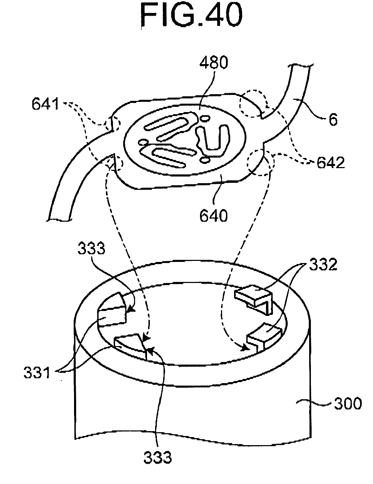

In response to this, a guiding wall 331 for positioning the board part 640 is provided in the inner circumference of the spacer 300 and a nail part 332 for a temporal fixation is provided at a position facing the guiding wall 331 as shown in FIG. 40, for example in the fifth embodiment. Each of the guiding wall 331 and the nail part 332 is preferably provided in pairs at positions where both sides of the flexible board 6 locate.

The guiding wall 331 has a plane 333 parallel with the axis of the spacer 300. By inserting the board part 640 to the inside of the spacer 300 while making a flat part 641 as an edge part at one side of the flexible board 640 in the board part 640 in direct contact with the plane 333, it is possible to determine the position of the board part 640 with respect to the spacer 300 easily. Besides, by making an edge part 642 at the other side of the flexible board 640 in the board part 640 engage with the nail part 332, it is possible to fix the flexible board 6 to the spacer 300 easily and temporarily. Here, it is preferable that a protruding amount of the nail part 332 to the inner diameter side is determined depending on a size of the board part 640.

### Sixth modification

Next, a modification example of the flexible board 6 will be explained.

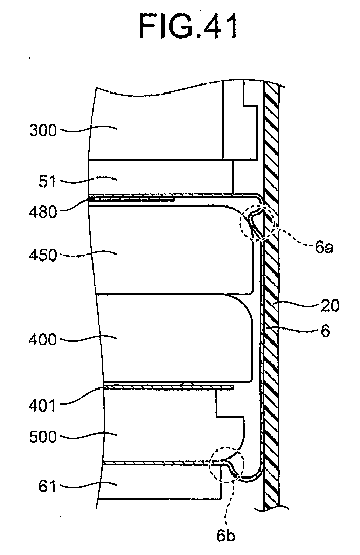

In assembling the capsule medical apparatus 1, the first block part 3, the battery unit 4, and the second block part 5 are housed in the inside of the casing 2 by folding the flexible board 6 connecting boards on which each functional unit is mounted. On this occasion, there is a possibility that the flexible board 6 is folded at a position that the operator does not intend (see unintended fold parts 6a and 6b) as shown in FIG. 41. In this case, there is a possibility that a pattern on the flexible board 6 gets broken or the flexible board 6 itself gets broken.

Therefore, the flexible board is manufactured in the following manner in the sixth modification so that the flexible board is folded at a position that the operator intends.

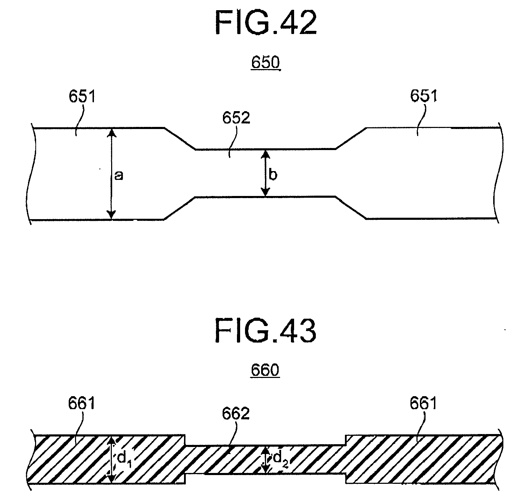

FIG. 42 is a top view of an example of manufacturing a flexible board. For example, a width b of a fold part 652 which is expected to be a fold part is narrower than a width a of a straight line part 651 which is expected to be kept straight when a flexible board 650 is arranged in the casing 2 (b < a) as shown in FIG. 42, for example.

FIG. 43 is a cross sectional view of another example of manufacturing a flexible board. A fold part 662 having a thickness d2 may be thinner than a straight line part 661 having a thickness dl of a flexible board 660 as shown in FIG. 43, for example. The flexible board 660 can be manufactured by making a thickness of a coverlay in the straight line part 661 (50 µm, for example) and a thickness of the coverlay in the fold part 662 (12.5 µm, for example) different from each other, for example.

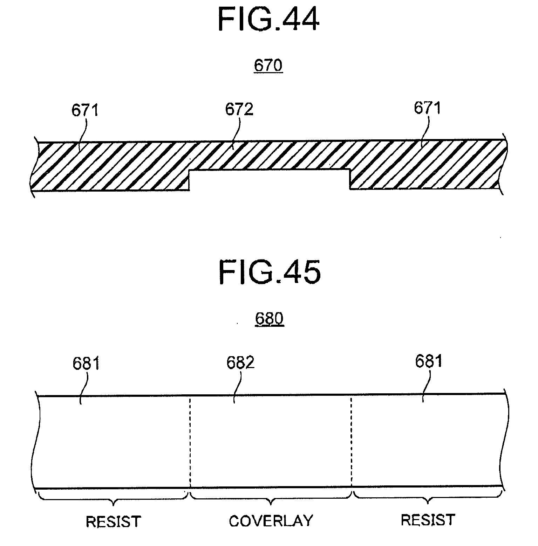

FIG. 44 is a cross sectional view of still another example of manufacturing a flexible board. In the case of making the fold part thinner than the straight line part of the flexible board, a thickness may be adjusted by making the thickness of the coverlay in a straight line part 671 and the thickness of the coverlay in a fold part 672 different by using only one side of a flexible board 670 as shown in FIG. 44, for example. In this case, it is possible to simplify a process of manufacturing the flexible board.

FIG. 45 is a top view of still another example of manufacturing a flexible board. A straight line part 681 of a flexible board 680 is formed by a resist formed of a thermoset resin and a fold part 682 is formed by a coverlay formed of a polyimide resin as shown in FIG. 45, for example. Since the polyimide resin has a higher flexibility than the thermoset resin, it becomes possible to surely fold the flexible board 680 in the fold part 682.

### EXPLANATIONS OF LETTERS OR NUMERALS

1 CAPSULE MEDICAL APPARATUS
2 CASING
3 FIRST BLOCK PART
4 BATTERY UNIT
5 SECOND BLOCK PART
6 FLEXIBLE BOARD
7 ADHESIVE AGENT
10 DOME PART
10a DOME HEMISPHERICAL PART
10b DOME GRASPING PART
10c DOME CYLINDRICAL PART
10d EDGE FACE
10e ROTATION REGULATING PART
10f INNER WALL RIB
20 CASE PART
20a CASE HEMISPHERICAL PART
20b CASE CYLINDRICAL PART
20c CASE FITTING PART
20d CASE GROOVE PART
20e CASE INNER WALL RIB
20f PLANAR PART
20g CASE EDGE PART
30 ILLUMINATION UNIT
30a OPENING
31 ILLUMINATION BOARD
32 ILLUMINATION ELEMENT
33 OPENING
40 IMAGING UNIT
41 IMAGING BOARD
42 IMAGING ELEMENT
42a LIGHT-RECEIVING SURFACE
43 CIRCUIT UNIT
43a IMAGING CONTROLLER
43b SIGNAL PROCESSOR
43c INTERNAL REGISTER
43d OSCILLATION CIRCUIT
44 OPENING
50 CONTROL UNIT
50G ELECTRONIC PART GROUP
51 CONTROL BOARD
51a LUG PART
52 REED SWITCH
53 POWER SOURCE IC
53a POWER SOURCE CONTROLLER
53b POWER SOURCE UNIT
53c ILLUMINATION DRIVER
54 MEMORY
55 CRYSTAL OSCILLATOR
60 WIRELESS COMMUNICATION UNIT
61 WIRELESS BOARD
61a LUG PART
62 TRANSMISSION ANTENNA
63 ELECTRONIC PART
64 PAD
200 OBJECTIVE LENS UNIT
201 FIRST LENS
201a, 202a, 203a FIRST LENS SURFACES
201b, 202b, 203b SECOND LENS SURFACES
201c LENS FRAME ABUTTING PART
201d, 202d, 203d LENS FRAME FITTING PARTS
201e APERTURE RECEIVER
202 SECOND LENS
202e LENS RECEIVER
202c APERTURE RECEIVING SURFACE
203 THIRD LENS
203c LENS ABUTTING PART
203e BOTTOM SURFACE
203f IMAGING ELEMENT ABUTTING PART
203g ABUTTING SURFACE
203h SIDE SURFACE AREA
203i POSITIONING LAND MARK PART
204 APERTURE
204a OPENING
205 LENS FRAME
205a, 205c, 205d LENS FITTING PART
205b LENS RECEIVER
205e GAP FORMING PART
205f POSITIONING LAND MARK PART
205g SPACER FITTING PART
205h SLOPE PART
205i ILLUMINATION BOARD INSTALLATION PART
205j CYLINDRICAL PART
205k SPACER ROTATION REGULATING PART
206a INSERTION OPENING
206b INCIDENT LIGHT OPENING
207 ADHESIVE AGENT
208 PROTRUDING PART
209 GUIDING PART
210 NAIL PART
300 SPACER
300a DOME FITTING PART
300b UPPER END PART
300c BOARD RECEIVER
310 FIRST SPACER DIVIDED BODY
310a, 320a LENS FRAME ENGAGEMENT PARTS
310b, 320b LENS FRAME ROTATION REGULATING PARTS
310c, 320c BOARD ENGAGEMENT PARTS
310d, 320d BOARD ROTATION REGULATING PARTS
310e, 320e BOSSES
310f, 320f FITTING HOLES
320 SECOND SPACER DIVIDED BODY
331 GUIDING WALL
332 NAIL PART
400 FIRST BATTERY LOT
401 POSITIVE ELECTRODE CONTACT POINT MEMBER
401a SPACER ENGAGEMENT PART
401b BOARD CONTACT PART
401c L-SHAPED BENT PART
401d CONVEX PART
402, 453 BATTERIES
403, 454 FASTENING MEMBERS
450 SECOND BATTERY LOT
451 INSULATION SHEET
451b EXTENSION PART
451c OPENING PART
451d SLOPE PART
452 CONTACT POINT PLATE
452a CONVEX PART
453a POSITIVE ELECTRODE SURFACE
453b NEGATIVE ELECTRODE SURFACE
461 BATTERY POSITIVE ELECTRODE CAN
462 NEGATIVE ELECTRODE
463 SEPARATOR
480 NEGATIVE POSITIVE CONTACT POINT SPRING
481 SPRING PART
482 PROTRUDING PART
483 CENTER PLANE PART
484 CUTOUT PART
490 SOLDER
500 SPACER
500a BOARD CONTACT POINT OPENING
500b POSITIVE ELECTRODE CONTACT POINT MEMBER ENGAGEMENT PART
500c POSITIVE ELECTRODE CONTACT POINT MEMBER POSITION REGULATING PART
500d BOARD RECEIVER
500e ROTATION REGULATING PART
500f ROUND CHAMFERING
510 FIRST SPACER DIVIDED BODY
510a, 520a WIRELESS BOARD ENGAGEMENT PARTS
510b, 520b WIRELESS BOARD ROTATION REGULATING PARTS
510c, 510c BOSSES
510d, 520d FITTING HOLES
520 SECOND SPACER DIVIDED BODY
610, 620, 630 ILLUMINATION BOARDS
650, 660, 670, 680 FLEXIBLE BOARDS
611, 621 EXTENSION PARTS
612, 622 LUG PARTS
623 TONGUE PART
631 CONCAVE PART
641 FLAT PART
642 EDGE PART
6a, 6b UNINTENDED FOLD PARTS
651, 661, 671, 681 STRAIGHT LINE PARTS
652, 662, 672, 682 FOLD PARTS
673 COVERLAY

## Claims

1. A capsule medical apparatus (1), comprising:
a casing (2) having a capsule shape, wherein the casing (2) is constituted by a dome part (10) provided with a dome hemispherical part (10a), and a case part (20) having a cylindrical part and a bottom;
first and second boards (41, 61) in at least one of which an imaging element (42) is provided and which are connected by a flexible board (6);
a first board retaining member (300) that retains the first board (41);
a second board retaining member (500) that retains the second board (61); and
a battery (4) arranged between the first and the second board retaining members, wherein
the first and the second board retaining members (300, 500) and the battery (4) are housed in an inside of the casing (2) in a state where the battery (4) is put between the first and the second board retaining members (300, 500) which retain the first and the second boards (41, 61), respectively, and
a groove part (20d) in which the flexible board (6) is arranged is formed on an inner wall of the casing (2),
**characterized in that**
a cross section of the groove (20d) perpendicular to a longitudinal direction of the groove part (20d) has a trapezoidal shape in which a width at a bottom part side of the groove part (20d) is narrower, and
the dome part (10) has a dome cylindrical part (10c) provided with a rotation regulating part (10e), and
the groove part (20d) is adapted to allow an engagement of r then rotation regulating part (10e) for fitting the dome part (10).

2. The capsule medical apparatus (1) according to claim 1, further comprising:
an imaging lens (201, 202, 203); and
a lens retaining frame (205) that retains the imaging lens (201, 202, 203), wherein
the first board retaining member (300) includes two members which are divided in a direction perpendicular to a longitudinal direction of the casing (2), determines a position of the lens retaining frame (205) in the longitudinal direction of the casing (2) and the direction perpendicular to the longitudinal direction by lodging at least a part of the lens retaining frame (205) therebetween, and determines a position of the first board (41) in the direction perpendicular to the longitudinal direction of the casing (2) by lodging the first board (41) therebetween.

3. The capsule medical apparatus (1) according to claim 1 or 2, wherein each of the first and the second board retaining members (300, 500) includes a position determining part that determines a position in a longitudinal direction and in a direction perpendicular to the longitudinal direction with respect to the casing (2) in the state of being housed in the inside of the casing (2).

4. The capsule medical apparatus (1) according to claim 1, further comprising:
a third board (51) on which a switch (52) that switches on and off a power source in response to an external magnetic field is mounted, wherein
the outer circumference of the cylindrical part of the case part (20) has a cylindrical shape,
a planar part (20f) is formed in a part of the outer circumference in the cylindrical part, and
an arranging direction of the third board (51) is determined depending on a position of the planar part (20f).

5. The capsule medical apparatus (1) according to claim 4, wherein the position of the groove part (20d) in a circumferential direction is different from the position of the planar part (20f).

6. The capsule medical apparatus (1) according to any one of claims 1 to 5, wherein
the dome part (10) engages with the first casing member (20) and serves as a lid of the first casing member (20),
the dome hemispherical part (10a) is formed of a transparent resin material and the dome part (10) further includes a further cylindrical part coupled to the dome hemispherical part (10a), and
a parting line generated in molding is provided in a boundary part between the further cylindrical part and the hemispherical part.

## Patentansprüche

1. Medizinisches Kapselgerät (1), das umfasst:
ein Gehäuse (2), das eine Kapselform hat, wobei das Gehäuse (2) durch ein Domteil (10), das mit einem halbkugelförmigen Domteil (10a) versehen ist, und ein Gehäuseteil (20), das ein zylindrisches Teil und einen Boden hat, gebildet wird;
erste und zweite Boards (41, 61), wobei in wenigstens einem davon ein Bildgebungselement (42) vorgesehen ist, und die durch ein flexibles Board (6) verbunden sind;
ein erstes Boardhalteelement (300), das das erste Board (41) hält;
ein zweites Boardhalteelement (500), das das zweite Board (61) hält; und
eine Batterie (4), die zwischen dem ersten und dem zweiten Boardhalteelement angeordnet ist, wobei
das erste und das zweite Boardhalteelement (300, 500) und die Batterie (4) in einem Innenraum des Gehäuses (2) in einem Zustand aufgenommen sind, in dem die Batterie (4) zwischen dem ersten und dem zweiten Boardhalteelement (300, 500) platziert ist, die das erste bzw. das zweite Board (41, 61) halten, und
ein Nutteil (20d), in dem das flexible Bord (6) angeordnet ist, an einer Innenwand des Gehäuses (2) ausgebildet ist,
**dadurch gekennzeichnet, dass**
ein Querschnitt der Nut (20d) senkrecht zu einer Längsrichtung des Nutteils (20d) eine Trapezoidform hat, bei der eine Breite an einer Bodenteilseite des Nutteils (20d) schmäler ist, und
das Domteil (10) ein zylindrisches Domteil (10c) hat, das mit einem Rotationssteuerungsteil (10e) versehen ist, und
das Nutteil (20d) dazu eingerichtet ist, ein Eingreifen des Rotationssteuerungsteils (10e) zu erlauben, um das Domteil (10) zu installieren.

2. Medizinisches Kapselgerät (1) gemäß Anspruch 1, das ferner umfasst:
eine Bildgebungslinse (201, 202, 203); und
einen Linsenhalterahmen (205), der die Bildgebungslinse (201, 202, 203) hält, wobei
das erste Boardhalteelement (300) zwei Elemente umfasst, die in einer Richtung senkrecht zu einer Längsrichtung des Gehäuses (2) getrennt sind, eine Position des Linsenhalterahmens (205) in der Längsrichtung des Gehäuses (2) und der Richtung senkrecht zu der Längsrichtung bestimmt, indem er zumindest einen Teil des Linsenhalterahmens (205) dazwischen befestigt, und eine Position des ersten Boards (41) in der Richtung senkrecht zu der Längsrichtung des Gehäuses (2) bestimmt, indem er das erste Board (41) dazwischen befestigt.

3. Medizinisches Kapselgerät (1) gemäß Anspruch 1 oder 2, bei dem jedes der ersten und zweiten Boardhalteelemente (300, 500) ein Positionsbestimmungsteil umfasst, das in einem in dem Innenraum des Gehäuses (2) angeordneten Zustand eine Position bezüglich des Gehäuses (2) in einer Längsrichtung und in einer Richtung senkrecht zu der Längsrichtung bestimmt.

4. Medizinisches Kapselgerät (1) gemäß Anspruch 1, das ferner umfasst:
ein drittes Board (51), auf dem ein Schalter (52) angebracht ist, der in Reaktion auf ein äußeres Magnetfeld eine Energiequelle an- und abschaltet, wobei
der Außenumfang des zylindrischen Teils des Gehäuseteils (20) eine zylindrische Form hat,
ein ebener Teil (20f) in einem Teil des Außenumfangs in dem zylindrischen Teil ausgebildet ist, und
eine Anordnungsrichtung des dritten Boards (51) in Abhängigkeit von einer Position des ebenen Teils (20f) bestimmt wird.

5. Medizinisches Kapselgerät (1) gemäß Anspruch 4, bei dem sich die Position des Nutteils (20d) in einer Umfangsrichtung von der Position des ebenen Teils (20f) unterscheidet.

6. Medizinisches Kapselgerät (1) gemäß einem der Ansprüche 1 bis 5, bei dem
das Domteil (10) in das erste Gehäuseelement (20) eingreift und als Deckel des ersten Gehäuseelements (20) dient,
das halbkugelförmige Domteil (10a) aus einem transparenten Harzmaterial besteht und das Domteil (10) ferner ein weiteres zylindrisches Teil umfasst, das mit dem halbkugelförmigen Domteil (10a) gekoppelt ist, und
eine bei der Formgebung erzeugte Trennlinie in einem Grenzteil zwischen dem weiteren zylindrischen Teil und dem halbkugelförmigen Teil vorgesehen ist.

## Revendications

1. Appareil médical de type capsule (1), comprenant :
un boîtier (2) ayant une forme de capsule, dans lequel le boîtier (2) est constitué par une partie (10) en dôme prévue avec une partie hémisphérique (10a) en dôme, et une partie (20) de boîtier ayant une partie cylindrique et un fond ;
des première et deuxième cartes (41, 61) dans au moins une desquelles un élément d'imagerie (42) est prévu et qui sont connectées par une carte flexible (6) ;
un premier élément (300) de maintien de carte qui maintient la première carte (41) ;
un deuxième élément (500) de maintien de carte qui maintient la deuxième carte (61) ; et
une batterie (4) agencée entre les premier et deuxième éléments de maintien de cartes, dans lequel
les premier et deuxième éléments (300, 500) de maintien de cartes et la batterie (4) sont logés dans un intérieur du boîtier (2) dans un état où la batterie (4) est mise entre les premier et deuxième éléments (300, 500) de maintien de cartes qui maintiennent les première et deuxième cartes (41, 61), respectivement, et
une partie (20d) de rainure dans laquelle la carte flexible (6) est agencée est formée sur une paroi intérieure du boîtier (2),
**caractérisé en ce que**
une section transversale de la rainure (20d) perpendiculaire à un sens longitudinal de la partie (20d) de rainure a une forme trapézoïdale dans laquelle une largeur sur un côté de partie de fond de la partie (20d) de rainure est plus étroite, et
la partie (10) en dôme a une partie cylindrique (10c) en dôme prévue avec une partie (10e) de régulation de rotation, et
la partie (20d) de rainure est adaptée à permettre un engagement de la partie (10e) de régulation de rotation pour un ajustement de la partie (10) en dôme.

2. Appareil médical de type capsule (1) selon la revendication 1, comprenant en outre :
une lentille d'imagerie (201, 202, 203) ; et
un cadre (205) de maintien de lentille qui maintient la lentille d'imagerie (201, 202, 203), dans lequel
le premier élément (300) de maintien de carte inclut deux éléments qui sont divisés dans un sens perpendiculaire à un sens longitudinal du boîtier (2), détermine une position du cadre (205) de maintien de lentille dans le sens longitudinal du boîtier (2) et le sens perpendiculaire au sens longitudinal en logeant au moins une partie du cadre (205) de maintien de lentille entre ceux-ci, et détermine une position de la première carte (41) dans le sens perpendiculaire au sens longitudinal du boîtier (2) en logeant la première carte (41) entre ceux-ci.

3. Appareil médical de type capsule (1) selon la revendication 1 ou 2, dans lequel chacun des premier et deuxième éléments (300, 500) de maintien de cartes inclut une partie de détermination de position qui détermine une position dans un sens longitudinal et dans un sens perpendiculaire au sens longitudinal par rapport au boîtier (2) dans l'état d'être logé dans l'intérieur du boîtier (2).

4. Appareil médical de type capsule (1) selon la revendication 1, comprenant en outre :
une troisième carte (51) sur laquelle un commutateur (52) qui active et désactive une source d'énergie en réponse à un champ magnétique externe est monté, dans lequel :
la circonférence extérieure de la partie cylindrique de la partie (20) de boîtier a une forme cylindrique,
une partie (20f) plane est formée dans une partie de la circonférence extérieure de la partie cylindrique, et
un sens d'agencement de la troisième carte (51) est déterminé en fonction d'une position de la partie (20f) plane.

5. Appareil médical de type capsule (1) selon la revendication 4, dans lequel la position de la partie (20d) de rainure dans un sens circonférentiel est différente de la position de la partie (20f) plane.

6. Appareil médical de type capsule (1) selon l'une quelconque des revendications 1 à 5, dans lequel
la partie (10) en dôme s'engage avec le premier élément (20) de boîtier et sert comme un couvercle du premier élément (20) de boîtier,
la partie hémisphérique (10a) en dôme est constituée d'une matière résineuse transparente et la partie (10) en dôme inclut en outre une autre partie cylindrique couplée à la partie hémisphérique (10a) en dôme, et
une ligne de séparation générée au moulage est prévue dans une partie de limite entre l'autre partie cylindrique et la partie hémisphérique.
